# EUROPÄISCHE PATENTANMELDUNG

(11) **EP 2 441 848 A1**
(43) Veröffentlichungstag der Anmeldung: **18.04.2012**
(21) Anmeldenummer: 10187316.4
(22) Anmeldetag: 12.10.2010
(51) Int. Cl.: C12Q 1/68

(54) **Markersequenzen für systemischer Lupus Erythematodes und deren Verwendung**

(71) Anmelder: Protagen AG, 44227 Dortmund (DE)
(72) Erfinder: Lüking, Angelika, 44892 Bochum (DE); Kowald, Axel, 44892 Bochum (DE); Müllner, Stefan, 40764 Langenfeld (DE); Scheer, Christian, 44149 Dortmund (DE); Schneider, Matthias, 40545 Düsseldorf (DE)
(74) Vertreter: Simandi, Claus

(57) **Zusammenfassung**

Die vorliegende Erfindung betrifft neue Markersequenzen für Systemischer Lupus erythematodes und deren diagnostische Verwendung samt einem Verfahren zum Screenen von potentiellen Wirkstoffen für Systemischer Lupus erythematodes - Erkrankungen mittels dieser Markersequenzen. Ferner betrifft die Erfindung eine diagnostische Vorrichtung enthaltend solche Markersequenzen für Systemischer Lupus erythematodes, insbesondere ein Proteinbiochip und dessen Verwendung.

## Beschreibung

Die vorliegende Erfindung betrifft neue Markersequenzen für Systemischer Lupus erythematodes und deren diagnostische Verwendung samt einem Verfahren zum Screenen von potentiellen Wirkstoffen für Systemischer Lupus erythematodes - Erkrankungen mittels dieser Markersequenzen. Ferner betrifft die Erfindung eine diagnostische Vorrichtung enthaltend solche Markersequenzen für Systemischer Lupus erythematodes, insbesondere ein Proteinbiochip und dessen Verwendung.

Proteinbiochips gewinnen eine zunehmende industrielle Bedeutung in der Analytik und Diagnostik sowie in der Pharmaentwicklung. Proteinbiochips haben sich als Screeninginstrumente etabliert.

Hierbei wird die schnelle und hochparallele Detektion einer Vielzahl spezifisch bindender Analysemoleküle in einem einzigen Experiment ermöglicht. Zur Herstellung von Proteinbiochips ist es erforderlich, die benötigten Proteine zur Verfügung zu haben. Hierzu haben sich insbesondere Protein-Expressionsbibliotheken etabliert. Die Hochdurchsatz-Klonierung von definierten offenen Leserahmen ist eine Möglichkeit (Heyman, J.A., Cornthwaite, J., Foncerrada, L., Gilmore, J.R., Gontang, E., Hartman, K.J., Hernandez, C.L., Hood, R., Hull, H.M., Lee, W.Y., Marcil, R., Marsh, E.J., Mudd, K.M., Patino, M.J., Purcell, T.J., Rowland, J.J., Sindici, M.L. and Hoeffler, J.P. (1999) Genome-scale cloning and expression of individual open reading frames using topoisomerase I-mediated ligation. Genome Res, 9, 383-392; Kersten, B., Feilner, T., Kramer, A., Wehrmeyer, S., Possling, A., Witt, I., Zanor, M.I., Stracke, R., Lueking, A., Kreutzberger, J., Lehrach, H. and Cahill, D.J. (2003) Generation of Arabidopsis protein chip for antibody and serum screening. Plant Molecular Biology, 52, 999-1010; Reboul, J., Vaglio, P., Rual, J.F., Lamesch, P., Martinez, M., Armstrong, C.M., Li, S., Jacotot, L., Bertin, N., Janky, R., Moore, T., Hudson, J.R., Jr., Hartley, J.L., Brasch, M.A., Vandenhaute, J., Boulton, S., Endress, G.A., Jenna, S., Chevet, E., Papasotiropoulos, V., Tolias, P.P., Ptacek, J., Snyder, M., Huang, R., Chance, M.R., Lee, H., Doucette-Stamm, L., Hill, D.E. and Vidal, M. (2003) C. elegans ORFeome version 1.1: experimental verification of the genome annotation and resource for proteome-scale protein expression. Nat Genet, 34, 35-41.; Walhout, A.J., Temple, G.F., Brasch, M.A., Hartley, J.L., Lorson, M.A., van den Heuvel, S. and Vidal, M. (2000) GATEWAY recombinational cloning: application to the cloning of large numbers of open reading frames or ORFeomes. Methods Enzymol, 328, 575-592). Allerdings hängt ein solcher Ansatz stark mit dem Fortschritt der Genom-Sequenzierungsprojekte und der Annotierung dieser Gensequenzen zusammen. Darüber hinaus ist die Bestimmung der exprimierten Sequenz aufgrund differenzieller Spleißvorgänge nicht immer eindeutig. Dieses Problem kann durch die Anwendung von cDNA-Expressionsbibliotheken umgangen werden (Büssow, K., Cahill, D., Nietfeld, W., Bancroft, D., Scherzinger, E., Lehrach, H. and Walter, G. (1998) A method for global protein expression and antibody screening on high-density filters of an arrayed cDNA library. Nucleic Acids Research, 26, 5007-5008; Büssow, K., Nordhoff, E., Lübbert, C., Lehrach, H. and Walter, G. (2000) A human cDNA library for high-throughput protein expression screening. Genomics, 65, 1-8; Holz, C., Lueking, A., Bovekamp, L., Gutjahr, C., Bolotina, N., Lehrach, H. and Cahill, D.J. (2001) A human cDNA expression library in yeast enriched for open reading frames. Genome Res, 11, 1730-1735; Lueking, A., Holz, C., Gotthold, C., Lehrach, H. and Cahill, D. (2000) A system for dual protein expression in Pichia pastoris and Escherichia coli, Protein Expr. Purif., 20, 372-378). Hierbei wird die cDNA eines bestimmten Gewebes in einen bakteriellen oder einen eukaryotischen Expressionsvektor, wie z.B. Hefe, einkloniert. Die für die Expression verwendeten Vektoren zeichnen sich im Allgemeinen dadurch aus, dass sie induzierbare Promotoren tragen, mit denen sich der Zeitpunkt der Proteinexpression steuern lässt. Darüber hinaus weisen Expressionsvektoren Sequenzen für so genannte Affinitätsepitope oder -proteine auf, die zum einen den spezifischen Nachweis der rekombinanten Fusions-Proteine mittels eines gegen das Affinitätsepitop gerichteten Antikörpers erlauben, zum anderen wird die spezifische Aufreinigung über Affinitätschromatographie (IMAC) ermöglicht.

Beispielsweise wurden die Genprodukte einer cDNA-Expressionsbibliothek aus humanem fötalem Hirngewebe in dem bakteriellen Expressionssystem Escherichia coli im Hochdichte-Format auf einer Membran angeordnet und konnten erfolgreich mit unterschiedlichen Antikörpern gescreent werden. Es konnte gezeigt werden, dass der Anteil an Volllänge-Proteinen bei mindestens 66% liegt. Die rekombinanten Proteine aus Expressionsbibliotheken konnten darüber hinaus im Hochdurchsatz exprimiert und aufgereinigt werden (Braun P., Hu, Y., Shen, B., Halleck, A., Koundinya, M., Harlow, E. and LaBaer, J. (2002) Proteome-scale purification of human proteins from bacteria. Proc Natl Acad Sci U S A, 99, 2654-2659; Büssow (2000) supra; Lueking, A., Horn, M., Eickhoff, H., Büssow, K., Lehrach, H. and Walter, G. (1999) Protein microarrays for gene expression and antibody screening. Analytical Biochemistry, 270, 103-111). Solche Proteinbiochips auf der Basis von cDNA-Expressionsbibliotheken sind insbesondere Gegenstand der WO 99/57311 und WO 99/57312.

Ferner sind neben Antigen-präsentierenden Proteinbiochips ebenfalls Antikörper-präsentierende Anordnungen beschrieben (Lal et al (2002) Antibody arrays: An embryonic but rapidly growing technology, DDT, 7, 143-149; Kusnezow et al. (2003), Antibody microarrays: An evaluation of production parameters, Proteomics, 3, 254-264).

Es besteht jedoch ein hohes Bedürfnis indikationsspezifische diagnostische Vorrichtungen, wie einen Proteinbiochip, bereitzustellen.

Die Aufgabe der vorliegenden Erfindung ist die Bereitstellung von verbesserten Markersequenzen und deren diagnostische Verwendung zur Behandlung von Systemischer Lupus erythematodes.

Die Bereitstellung von spezifischen Markersequenzen erlaubt eine sichere Diagnose und Stratifizierung von Patienten mit Systemischer Lupus erythematodes, insbesondere mittels eines Proteinbiochips.

Daher betrifft die Erfindung die Verwendung von Markersequenzen zur Diagnose von Systemischer Lupus erythematodes, wobei mindestens eine Markersequenz einer cDNA ausgewählt aus der Gruppe SEQ 1 - 716 oder jeweils ein dafür kodierendes Protein oder jeweils einer Teilsequenz oder Fragment davon (nachstehend: erfindungsgemäße Markersequenzen) an oder von einem zu untersuchenden Patienten bestimmt wird. Die erfindungsgemäßen Markersequenzen konnten mittels differentiellem Screenen von Proben und zwar gesunder Probanden mit Patientenproben mit Systemischer Lupus erythematodes identifiziert werden.

Hierbei konnte erstmals mittels Proteinbiochips (siehe Beispiele) diese erfindungsgemäßen Markersequenzen identifiziert werden.

Der Begriff "*Systemischer Lupus erythematodes (SLE*) "betrifft eine systemische Autoimmunerkrankung aus der Gruppe der Kollagenosen. Besonders charakteristisch für SLE (systemischer Lupus erythematodes) ist das so genannte Schmetterlingserythem.

Diagnosekriterien sind:
1. Schmetterlingserythem
2. diskoide Hautveränderungen
3. Lichtempfindlichkeit
4. Schleimhautulzera (im Allgemeinen schmerzlos)
5. Arthritis in mindestens zwei Gelenken
6. Serositis (Pleuritis od. Perikarditis)
7. Nierenbeteiligung (Proteinurie >0,5 g/d od. Zylinder)
8. ZNS-Beteiligung (Krämpfe od. Psychose)
9. hämatologische Befunde (hämolyt. Anämie, Leuko- od.
   Thrombopenie)
10. immunologische Befunde:
   Anti-dsDNA-Antikörper, Anti-Sm-Antikörper, Antikardiolipin-Antikörper;
11. antinukleäre Antikörper ohne Einnahme Lupus erythematodes auslösender Medikamente;

Auswertung: bei vier (drei) positiven Befunden gilt die Diagnose als sicher (wahrscheinlich) (Definition z.B. nach Pschyrembel, de Gruyter, 261. Auflage (2007), Berlin).

Erfindungswesentlich ist, dass die Proben nicht aus üblichen Blutbanken entnommen werden, sondern von SLE-Patienten sorgfältig ausgewählt wurden, die z.B. HIV und HCV negativ sind und insbesondere auf Infektionskrankheiten getestet wurden. Das aufwändige Probeselektionsverfahren erlaubt z.B. eine hinreichende vorteilhafte Abgrenzung von Erkrankungen mit SLE ähnlichen Symptomen. Weiterhin werden falsch-positive Ergebnisse ausgeschlossen, zum einen aufgrund der strengen bioinformatorischen Auswertung (siehe Beispiele).

Weiterhin erfolgt die Herstellung der Proteinbiochips mittels Normalisierung von mindestens 1.000, vorzugsweise 2.000 verschiedenen oder mehr Autoantigenen des Menschen, die nicht indikationsspezifisch für Systemischer Lupus erythematodes sind. Solche Autoantigene können z.B. aus anderen Körperflüssigkeiten von Patienten anderer Krankheiten gewonnen werden (z.B. Pankreaskrebs, Rheumatoide Arthritis, Prostata etc.).

Daher betrifft die Erfindung auch solche erfindungsgemäße indikationsspezifische Proteinbiochips zur Diagnose von Systemischer Lupus erythematodes, wobei in einem weiteren Verifizierungsschritt, die auf dem Proteinbiochip repräsentierte Proteine mit Autoantikörper aus Nicht-Systemischer Lupus erythematodes Patienten normalisiert werden und auf diese Weise falsch-positive Proteine entfernt werden können. Verbleibende nicht-falsch-positive Proteine können auf einem Proteinbiochip neu zusammengestellt werden, so genanntes Rearraying. Dies erlaubt ebenfalls den Ausschluss von Autoantikörpern, die auf E. coli positiv sind. Dies ist eine weitere qualitative Verbesserung, da Autoantikörper ausgeschlossen werden können, die z.B. gegen E. coli Darmbakterien im Menschen gerichtet sind. Infolge dessen können vorteilhaft bei einem verbesserten Signal/Rausch Verhältnis neue Markersequenzen identifiziert werden.

In einer weiteren bevorzugten Ausführungsform werden daher mindestens 2 bis 5 oder 10, vorzugsweise 30 bis 50 Markersequenzen oder 50 bis 100 oder mehr Markersequenzen an oder von einem zu untersuchenden Patienten bestimmt.

In einer weiteren Ausführungsform der Erfindung können die erfindungsgemäßen Markersequenzen ebenfalls mit bekannten Biomarkern für diese Indikation kombiniert, ergänzt oder erweitert werden.

In einer bevorzugten Ausführungsform erfolgt die Bestimmung der Markersequenzen außerhalb des menschlichen Körpers und die Bestimmung erfolgt in einer ex vivo / in vitro Diagnose.

In einer weiteren Ausführungsform der Erfindung betrifft die Erfindung die Verwendung von Markersequenzen als Diagnostika, wobei mindestens eine Markersequenz einer cDNA ausgewählt aus der Gruppe SEQ 1 - 716 oder jeweils ein dafür kodierendes Protein oder jeweils einer Teilsequenz oder Fragment davon ist.

Ferner betrifft die Erfindung ein Verfahren zur Diagnose von Systemischer Lupus erythematodes, wobei a.) mindestens eine Markersequenz einer cDNA ausgewählt aus der Gruppe SEQ 1 - 716 oder jeweils ein dafür kodierendes Protein oder jeweils einer Teilsequenz oder Fragment davon auf einem festen Träger aufgebracht wird und b.) mit Körperflüssigkeit oder Gewebeauszug eines Patienten in Kontakt gebracht wird und c.) der Nachweis einer Wechselwirkung der Körperflüssigkeit oder Gewebeauszug mit den Markersequenzen aus a.) erfolgt.

Daher betrifft die Erfindung ebenfalls Diagnostika zur Diagnose von Systemischer Lupus erythematodes jeweils ausgewählt aus der Gruppe SEQ 1 - 716 oder jeweils ein dafür kodierendes Protein oder jeweils einer Teilsequenz oder Fragment davon.

Der Nachweis einer solchen Wechselwirkung kann beispielsweise durch eine Sonde, insbesondere durch einen Antikörper erfolgen.

Daher betrifft die Erfindung ebenfalls die Aufgabe eine diagnostische Vorrichtung oder einen Assay, insbesondere einen Proteinbiochip, bereitzustellen, der für die Systemischer Lupus erythematodes eine Diagnose oder Untersuchung erlaubt.

Ferner betrifft die Erfindung ein Verfahren zum Stratifizieren, insbesondere zur Risikostratifizierung und / oder Therapiesteuerung eines Patienten mit Systemischer Lupus erythematodes, wobei mindestens eine Markersequenz einer cDNA ausgewählt aus der Gruppe SEQ 1 - 716 oder jeweils ein dafür kodierendes Protein an einem zu untersuchenden Patienten bestimmt wird.

Ferner umfasst ist die Stratifizierung der Patienten mit Systemischer Lupus erythematodes in neue oder etablierte Subgruppen der Systemischer Lupus erythematodes, sowie die sinnvolle Auswahl von Patientengruppen für die klinische Entwicklung von neuen Therapeutika. Der Begriff Therapiesteuerung umfasst ebenfalls die Einteilung von Patienten in Responder und Nicht-Responder bezüglich einer Therapie oder dessen Therapieverlauf.

"Diagnose" im Sinne dieser Erfindung bedeutet die positive Feststellung der Systemischer Lupus erythematodes mittels der erfindungsgemäßen Markersequenzen sowie die Zuordnung der Patienten zur Erkrankung an Systemischer Lupus erythematodes. Der Begriff der Diagnose umfasst die medizinische Diagnostik und diesbezügliche Untersuchungen, insbesondere die in-vitro Diagnostik und Labordiagnostik, ebenfalls Proteomics und Nukleinsäureblots. Weitere Untersuchungen können zur Absicherung und zum Ausschluss anderer Krankheiten vonnöten sein. Daher umfasst der Begriff Diagnose ebenfalls die Differentialdiagnose von Systemischer Lupus erythematodes mittels der erfindungsgemäßen Markersequenzen sowie die Prognose der Systemischer Lupus erythematodes.

"Stratifizieren (auch: Stratifikation) oder Therapiesteuerung" im Sinne dieser Erfindung bedeutet, dass das erfindungsgemäße Verfahren Entscheidungen zur Behandlung und Therapie des Patienten erlaubt, sei es Hospitalisierung des Patienten, Einsatz, Wirkung und / oder Dosierung eines oder mehrerer Arzneimittel, eine therapeutische Maßnahme oder die Überwachung eines Krankheitsverlaufes sowie Therapieverlauf bzw. Ätiologie oder Klassifizierung einer Erkrankung, z.B. in einen neuen oder bestehenden Subtyp oder die Differenzierung von Krankheiten und dessen Patienten.

In einer weiteren Ausführungsform der Erfindung umfasst der Begriff "Stratifizierung" insbesondere die Risikostratifizierung mit der Prognose eines "outcome" eines nachteiligen gesundheitlichen Ereignisses.

Im Rahmen dieser Erfindung wird unter "Patient" ein beliebiger Proband - Mensch oder Säugetier - verstanden, mit der Maßgabe, dass der Proband auf Systemischer Lupus erythematodes untersucht wird.

Der Begriff "Markersequenzen" im Sinne dieser Erfindung bedeutet, dass die cDNA oder das jeweils daraus erhältliche Polypeptid oder Protein signifikant für Systemischer Lupus erythematodes sind. Beispielsweise können die cDNA oder das jeweils daraus erhältliche Polypeptid oder Protein eine Wechselwirkung mit Substanzen aus der Körperflüssigkeit oder Gewebeauszug eines Patienten mit Systemischer Lupus erythematodes aufweisen (z.B. Antigen (Epitop) / Antikörper (Paratop) Wechselwirkung). Im Sinne der Erfindung bedeutet "wobei mindestens eine Markersequenz einer cDNA ausgewählt aus der Gruppe SEQ 1 - 716 oder jeweils ein dafür kodierendes Protein oder jeweils einer Teilsequenz oder Fragment davon an einem zu untersuchenden Patienten bestimmt wird", dass eine Wechselwirkung zwischen der Körperflüssigkeit oder Gewebeauszuges eines Patienten und den erfindungsgemäßen Markersequenzen nachgewiesen wird. Eine solche Wechselwirkung ist z.B. eine Bindung, insbesondere eine bindende Substanz an mindestens einer erfindungsgemäßen Markersequenz oder im Fall einer cDNA die Hybridisierung mit einer geeigneten Substanz unter gewählten Bedingungen, insbesondere stringenten Bedingungen (z.B. wie üblich definiert in J. Sambrook, E.F. Fritsch, T. Maniatis (1989), Molecular cloning: A laboratory manual, 2nd Edition, Cold Spring Habor Laboratory Press, Cold Spring Habor, USA oder Ausubel, "Current Protocols in Molecular Biology", Green Publishing Associates and Wiley Interscience, N.Y. (1989)). Ein Beispiel für stringente Hybridisierungsbedingungen ist: Hybridisierung in 4 x SSC bei 65° C (alternativ in 50% Formamid und 4 X SSC bei 42° C), gefolgt von mehreren Waschschritten in 0,1 x SSC bei 65°C für insgesamt etwa eine Stunde. Ein Beispiel für wenig stringente Hybridisierungsbedingungen ist Hybridisierung in 4 x SSC bei 37° C, gefolgt von mehreren Waschritten in 1 x SSC bei Raumtemperatur.

Solche Substanzen sind erfindungsgemäß Bestandteil einer Körperflüssigkeit, insbesondere Blut, Vollblut, Blutplasma, Blutserum, Patientenserum, Urin, Cerebrospinalflüssigkeit, Synovialflüssigkeit oder eines Gewebeauszuges des Patienten.

In einer weiteren Ausführungsform der Erfindung können jedoch die erfindungsgemäßen Markersequenzen in einer signifikant höheren oder niedrigeren Expressionsrate oder Konzentration vorliegen, dass auf die Systemischer Lupus erythematodes hinweist. Hierbei wird mittels Proteomics oder Nukleinsäureblots die relativen Expressionsraten krank / gesund der erfindungsgemäßen Markersequenzen für Systemischer Lupus erythematodes bestimmt.

Die Markersequenzen verfügen in einer weiteren Ausführungsform der Erfindung über ein Erkennungssignal, welches an die zu bindende Substanz adressiert ist (z.B. Antikörper, Nukleinsäure). Erfindungsgemäß bevorzugt ist für ein Protein das Erkennungssignal ein Epitop und / oder Paratop und / oder Hapten und für eine cDNA eine Hybridisierungs- oder Bindungsregion.

Die erfindungsgemäßen Markersequenzen sind ebenfalls Gegenstand der Tabelle A und können durch den jeweilig zitierten Datenbankeintrag (auch mittels Internet: http://www.ncbi.nlm.nih.gov/) eindeutig identifiziert werden (siehe in Tabelle A: dort Accession No.), siehe ebenfalls das zugehörige Sequenzprotokoll.

Daher betrifft die Erfindung ebenfalls die Volllängesequenzen der erfindungsgemäßen Marker und zwar wie in Tabelle A über den bekannten Datenbankeintrag definiert, nachstehend SEQ 1a-716a (cDNA) genannt.

Weiterhin umfasst sind daher ebenfalls analoge Ausführungsformen von SEQ 1a-716a zu den Markersequenzen SEQ 1-716, wie z.B. in den Ansprüchen dargelegt, da die erfindungsgemäßen SEQ 1-716 wiederum Teilsequenzen, zumindest mit hoher Homologie, darstellen. Die spezifischen Markersequenzen SEQ 1-716 sind jedoch erfindungsgemäß bevorzugt.

Erfindungsgemäß umfassen die Markersequenzen auch solche Modifikationen der cDNA-Sequenz und der entsprechenden Aminosäuresequenz, wie chemische Modifikation, wie Citrullinierung, Acetylierung, Phosphorylierung, Glykosilierung oder polyA-Strang und weiteren dem Fachmann einschlägig bekannte Modifikationen.

In einer weiteren Ausführungsform der Erfindung sind ebenfalls Teilsequenzen oder Fragmente der erfindungsgemäßen Markersequenzen umfasst. Insbesondere solche Teilsequenzen, die eine Identität von 95%, 90 %, insbesondere 80% oder 70 % mit den erfindungsgemäßen Markersequenzen aufweisen. Teilsequenzen sind ebenfalls solche Sequenzen, die 50 bis 100 Nukleotide, 70-120 Nukleotide einer Sequenz der SEQ 1-716 aufweisen, oder davon erhältliche Peptide.

"Teilsequenzen oder Fragmente" der erfindungsgemäßen Markersequenzen sind funktionell definiert und umfasst solche Sequenzen, die die gleiche erfindungsgemäße diagnostische Funktion aufweisen.

In einer weiteren Ausführungsform kann die jeweilige Markersequenz in unterschiedlichen Mengen in einen oder mehreren Bereichen auf einem festen Träger repräsentiert sein. Dies erlaubt eine Variation der Sensitivität. Die Bereiche können jeweils eine Gesamtheit von Markersequenzen aufweisen, d.h. eine genügende Zahl an verschiedenen Markersequenzen, insbesondere 2 bis 5 oder 10 oder mehr und ggfs. weiteren Nukleinsäuren und/oder Proteinen, insbesondere Biomarker. Bevorzugt sind jedoch mindestens 96 bis 25.000 (numerisch) oder mehr aus verschiedenen oder gleichen Markersequenzen und weiteren Nukleinsäuren und/oder Proteinen, insbesondere Biomarker. Weiterhin bevorzugt sind mehr als 2.500, besonders bevorzugt 10.000 oder mehr verschiedene oder gleiche Markersequenzen und ggfs. weiteren Nukleinsäuren und/oder Proteinen, insbesondere Biomarker.

Ein weiterer Gegenstand der Erfindung betrifft eine Anordnung von Markersequenzen enthaltend mindestens eine Markersequenz einer cDNA ausgewählt aus der Gruppe SEQ 1 - 716 oder jeweils ein dafür kodierendes Protein. Vorzugsweise enthält die Anordnung mindestens 2 bis 5 oder 10, vorzugsweise 30 bis 50 Markersequenzen oder 50 bis 100 oder mehr Markersequenzen.

Im Rahmen dieser Erfindung bedeutet "Anordnung" synonym "Array" und sofern dieser "Array" zur Identifizierung von Substanzen an Markersequenzen verwendet wird, ist hierunter ein "Assay" oder eine diagnostische Vorrichtung zu verstehen. In einer bevorzugten Ausführungsform ist die Anordnung derart gestaltet, dass die auf der Anordnung repräsentierten Markersequenzen in Form eines Gitters auf einem festen Träger vorliegen. Ferner sind solche Anordnungen bevorzugt, die eine hochdichte (high-density) Anordnung von Proteinbindern erlauben und die Markersequenzen gespottet werden. Solche hochdichte gespotteten Anordnungen sind beispielsweise in der WO 99/57311 und WO 99/57312 offenbart und können vorteilhaft in einem robotergestützten automatisierten High-Throughput Verfahren zur Anwendung kommen.

Im Rahmen dieser Erfindung umfasst jedoch der Begriff "Assay" oder diagnostische Vorrichtung ebenfalls solche Ausführungsformen einer Vorrichtung, wie ELISA, Bead-based Assay, Line Assay, Western Blot, immunchromatographische Verfahren (z.B. so genannte Lateral Flow Immunoassays) oder ähnliche immunologische Single- oder Multiplex-Nachweisverfahren. Ein Proteinbiochip im Sinne dieser Erfindung ist die systematische Anordnung von Proteinen auf einem festen Träger.

Die Markersequenzen der Anordnung sind auf einen festen Träger fixiert, vorzugsweise jedoch gespottet oder immobilisiert gar aufgedruckt, d.h. reproduzierbar aufgebracht. Ein oder mehrere Markersequenzen können mehrfach in der Gesamtheit aller Markersequenzen präsent sein und in unterschiedlichen Mengen bezogen auf einen Spot vorliegen. Ferner können die Markersequenzen auf dem festen Träger standardisiert sein (z.B. mittels serieller Verdünnungsreihen von z.B. Humanglobulinen als interne Kalibratoren zur Datennormalisierung und quantitativen Auswertung).

Daher betrifft die Erfindung einen Assay oder Proteinbiochip bestehend aus einer Anordnung enthaltend erfindungsgemäße Markersequenzen.

In einer weiteren Ausführungsform liegen die Markersequenzen als Clone vor. Solche Clone können beispielsweise mittels einer erfindungsgemäßen cDNA-Expressionsbibliothek erhalten werden (Büssow et al. 1998 (supra)). In einer bevorzugten Ausführungsform werden solche Expressionsbibliotheken enthaltend Clone mittels Expressionsvektoren aus einer exprimierenden cDNA Bibliothek bestehend aus den cDNA Markersequenzen erhalten. Diese Expressionsvektoren enthalten vorzugsweise induzierbare Promotoren. Die Induktion der Expression kann z.B. mittels eines Induktors, solche wie IPTG, erfolgen. Geeignete Expressionsvektoren sind beschrieben in Terpe et al. (Terpe T Appl Microbiol Biotechnol. 2003 Jan; 60(5):523-33).

Expressionsbibliotheken sind dem Fachmann bekannt, diese können nach Standardwerken, wie Sambrook et al, "Molecular Cloning, A laboratory handbook, 2nd edition (1989), CSH press, Cold Spring Harbor, New York hergestellt werden. Weiterhin bevorzugt sind solche Expressionsbibliotheken, die gewebespezifisch sind (z.B. humanes Gewebe, insbesondere humane Organe). Ferner sind erfindungsgemäß ebenfalls solche Expressionsbibliotheken mit eingeschlossen, die mittels exontrapping erhalten werden können. Statt Expressionsbibliothek kann synonym von einer Expressionsbank gesprochen werden. Weiterhin bevorzugt sind Proteinbiochips oder entsprechende Expressionsbibliotheken, die keine Redundanz aufweisen (so genannte: Uniclone®-Bibliothek) und nach den Lehren der WO 99/57311 und WO 99/57312 beispielsweise hergestellt werden können. Diese bevorzugten Uniclone- Bibliotheken weisen einen hohen Anteil an nicht-fehlerhaften vollständig exprimierten Proteinen einer cDNA-Expressionsbibliothek auf.

Im Rahmen dieser Erfindung können die Clone ebenfalls nicht abschließend solche sein, wie transformierte Bakterien, rekombinante Phagen oder transformierte Zellen von Säugern, Insekten, Pilzen, Hefen oder Pflanzen.

Die Clone werden auf einen festen Träger fixiert, gespottet oder immobilisiert.

Daher betrifft die Erfindung eine Anordnung, wobei die Markersequenzen als Clone vorliegen.

Zusätzlich können die Markersequenzen in der jeweiligen Form in Form eines Fusionsproteins vorliegen, welches beispielsweise mindestens ein Affinitätsepiptop oder "Tag" enthält. Der Tag kann ein solcher sein wie wie c-myc, His-Tag, Arg-tag, FLAG, alkalische Phosphatase, V5-Tag, T7-Tag oder Strep-Tag, HAT-tag, NusA, S-tag, SBP-tag, Thioredoxin, DsbA, ein Fusionsprotein, vorzugsweise eine Cellulose-bindende Domäne, grünfluoreszierendes Protein, Maltose bindendes Protein, calmodulin-bindendes Protein, Glutathione S-transferase oder lacZ enthalten.

In sämtlichen Ausführungsformen umfasst der Begriff "fester Träger" Ausführungen wie einen Filter, eine Membran, ein magnetisches oder Fluorophor-markiertes Kügelchen, ein Silizium-Wafer, Glas, Metall, Kunststoff, ein Chip, ein massenspektrometrisches Target oder eine Matrix. Ein Filter ist jedoch erfindungsgemäß bevorzugt.

Als Filter ist weiterhin PVDF, Nitrocellulose oder Nylon bevorzugt (z.B. Immobilon P Millipore, Protran Whatman, Hybond N+ Amersham).

In einer weiteren bevorzugten Ausführungsform der erfindungsgemäßen Anordnung entspricht diese einem Gitter, dass die Größenordnung einer Mikrotiterplatte (8-12 Wells Streifen, 96 Wells, 384 Wells oder mehr), eines Silizium-Wafers, eines Chips, eines massenspektrometrischen Targets oder einer Matrix besitzt.

In einer weiteren Ausführungsform betrifft die Erfindung einen Assay oder Proteinbiochip zum Identifizieren und Charakterisieren einer Substanz für Systemischer Lupus erythematodes, dadurch gekennzeichnet, dass eine erfindungsgemäße Anordnung oder Assay mit a.) mindestens einer zu untersuchenden Substanz in Kontakt gebracht wird und b.) ein Bindungserfolg nachgewiesen wird.

Ferner betrifft die Erfindung ein Verfahren zum Identifizieren und Charakterisieren einer Substanz für Systemischer Lupus erythematodes, dadurch gekennzeichnet, dass eine erfindungsgemäße Anordnung oder Assay mit a.) mindestens einer zu untersuchenden Substanz in Kontakt gebracht wird und b.) ein Bindungserfolg nachgewiesen wird.

Die zu untersuchende Substanz kann ein beliebiges natives oder nicht-natives Biomolekül, ein synthetisches chemisches Molekül, eine Mischung oder eine Substanzbibliothek sein.

Nachdem die zu untersuchende Substanz eine Markersequenz kontaktiert, erfolgt die Auswertung des Bindungserfolges, die beispielsweise unter Verwendung mit handelsüblicher Image-Analyse Software (GenePix Pro (Axon Laboratories), Aida (Raytest), ScanArray (Packard Bioscience) erfolgt.

Die Visualisierung erfindungsgemäßer Protein-Protein-Wechselwirkungen (z.B. Protein an Markersequenz, wie Antigen/Antikörper) oder entsprechende "Mittel zum Nachweis des Bindungserfolges" kann beispielsweise mittels Fluoresenzmarkierung, Biotiniylierung, Radio-Isotopen-Markierung oder kolloidale Gold- oder Latex-Partikel-Markierung in üblicher Weise erfolgen. Ein Nachweis von gebundenen Antikörpern erfolgt mit Hilfe von sekundären Antikörpern, die mit handelsüblichen Reportermolekülen markiert sind (z.B. Cy-, Alexa-, Dyomics, FITC- oder ähnliche Fluoreszenzfarbstoffe, , kolloidale Gold- oder Latex-Partikel), oder mit Reporter-Enzymen wie alkalischer Phosphatase, Meerrettichperoxidase, usw. und den entsprechenden colorimetrischen, fluoreszenten oder chemolumineszenten Substraten. Eine Auslesung erfolgt z.B. mittels eines Microarray-Laserscanners, einer CCD-Kamera oder visuell.

In einer weiteren Ausführungsform betrifft die Erfindung ein Arzneimittel / Wirkstoff oder Prodrug für Systemischer Lupus erythematodes entwickelt und erhältlich durch den Einsatz des erfindungsgemäßen Assays oder Proteinbiochip.

Daher betrifft die Erfindung ebenfalls die Verwendung einer erfindungsgemäßen Anordnung oder einem Assay zum Screenen von Wirkstoffen für Systemischer Lupus erythematodes.

Daher betrifft die Erfindung in einer weiteren Ausführungsform ebenfalls ein Target zur Behandlung und Therapie von Systemischer Lupus erythematodes, jeweils ausgewählt aus der Gruppe SEQ 1 - 716 oder jeweils ein dafür kodierendes Protein.

In einer weiteren Ausführungsform betrifft die Erfindung ebenfalls die Verwendung der erfindungsgemäßen Markersequenzen, vorzugsweise in Form einer Anordnung, als Affinitätsmaterial zur Durchführung einer Apherese bzw. iwS. einer Blutwäsche, wobei Substanzen aus Körperflüssigkeiten eines Patienten mit Systemischer Lupus erythematodes, wie Blut oder Plasma, an die erfindungsgemäßen Markersequenzen binden und folglich der Körperflüssigkeit selektiv entzogen werden können.

Beispiele und Figuren:
Zehn oder mehr Patientenproben wurden individuell gegen eine cDNA Expressionsbibliothek gescreent. Die Systemischer Lupus erythematodes -spezifischen Expressionsklone wurden ermittelt durch einen Vergleich mit zehn oder mehr gesunden Proben. Die Identität der Markersequenzen wurde durch DNA-Sequenzierung ermittelt.

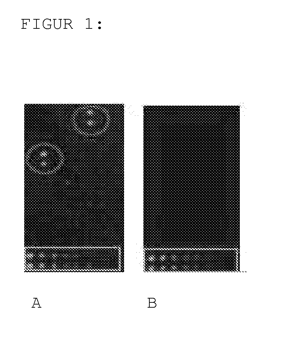

In Figur 1 wird das differentielle Screenen zwischen zwei Proteinbiochips aus jeweils einer cDNA-Expressionsbank eines Patienten und einem gesunden Probanden gezeigt. Die differentiellen Clone werden mittels Fluoresenzmarkierung nachgewiesen und bioinformatorisch ausgewertet.

Im Rahmen der Biomarkeridentifizierung werden verschiedene bioinformatische Analysen durchgeführt. Für jedes Serum werden mittels Microarray Reaktivitäten gegen ca. 2000 unterschiedliche Antigene gemessen. Diese Daten werden für ein Ranking der gespotteten Antigene bzgl. ihrer Differenzierungsfähigkeit zwischen gesunden und erkrankten Seren benutzt. Diese Auswertung wird mittels des nicht parametrischen Mann-Whitney Tests auf normalisierten Intensitätsdaten durchgeführt. Zur Normalisierung wird ein interner Standard benutzt, der auf jedem Chip mitgespottet wird. Da für jedes Antigen ein p-Wert berechnet wird, werden Methoden zur Korrektur des multiples Testens eingesetzt. Als sehr konservativer Ansatz wird eine Bonferroni Korrektur durchgeführt und zusätzlich wird die weniger restriktive False Discovery Rate (FDR) nach Benjamini & Hochberg berechnet.

Desweiteren werden die Daten zur Klassifikation der Seren benutzt. Hierbei kommen unterschiedliche multivariate Methoden zum Einsatz. Dies sind Methoden aus den statistischen Lernverfahren wie Support Vector Machines (SVM), Neuronale Netze oder Klassifikationsbäume, sowie eine Schwellenwertmethode, welche sowohl zur Klassifikation als auch zur visuellen Repräsentation der Daten geeignet ist.

Zur Vermeidung von Overfitting wird eine 10fache Cross-Validierung der Daten durchgeführt.

**Tabelle A: (gi Accession Nummer mit Geltung vom 1.10.2010)**

| SEQ 1a-716a | Name |
|---|---|
| gil66932947 | alpha-2-macroglobulin precursor [Homo sapiens] |
| gil55743075 | angio-associated, migratory cell protein [Homo sapiens] |
| gil45446740 | ATP-binding cassette, sub-family A, member 2 isoform a [Homo sapiens] |
| gil21536349 | amiloride-sensitive cation channel 2, neuronal isoform b [Homo sapiens] |
| gil4501867 | aconitase 2 precursor [Homo sapiens] |
| gil4501885 | beta actin [Homo sapiens] |
| gil4501887 | actin, gamma 1 propeptide [Homo sapiens] |
| gil29826323 | adducin 1 (alpha) isoform c [Homo sapiens] |
| gil194353970 | alpha-2A-adrenergic receptor [Homo sapiens] |
| gil148539876 | beta adrenergic receptor kinase 1 [Homo sapiens] |
| gil4501989 | alpha-fetoprotein precursor [Homo sapiens] |
| gil156523970 | alpha-2-HS-glycoprotein [Homo sapiens] |
| gil4502027 | albumin preproprotein [Homo sapiens] |
| gil4557305 | aldolase A [Homo sapiens] |
| gil32967601 | ankyrin 3 isoform 1 [Homo sapiens] |
| gil55749577 | solute carrier family 25 (mitochondrial carrier; adenine nucleotide translocator), member 4 [Homo sapiens] |
| gil156071459 | solute carrier family 25, member 5 [Homo sapiens] |
| gil156071462 | solute carrier family 25, member A6 [Homo sapiens] |
| gil4502131 | amyloid beta A4 precursor protein-binding, family B, member 1 isoform E9 [Homo sapiens] |
| gil4885065 | amyloid precursor-like protein 1 isoform 2 precursor [Homo sapiens] |
| gil4502201 | ADP-ribosylation factor 1 [Homo sapiens] |
| gil4502209 | ADP-ribosylation factor 5 [Homo sapiens] |
| gil33469974 | activating transcription factor 4 [Homo sapiens] |
| gil49574491 | Na+/K+ -ATPase beta 2 subunit [Homo sapiens] |
| gil4757810 | ATP synthase, H+ transporting, mitochondrial F1 complex, alpha subunit precursor [Homo sapiens] |
| gil32189394 | ATP synthase, H+ transporting, mitochondrial F1 complex, beta subunit precursor [Homo sapiens] |
| gil50593533 | ATP synthase, H+ transporting, mitochondrial F0 complex, subunit C2 isoform a precursor [Homo sapiens] |
| gil4502313 | ATPase, H+ transporting, lysosomal, V0 subunit c [Homo sapiens] |
| gil20336205 | transcriptional regulator ATRX isoform 2 [Homo sapiens] |
| gil4502337 | alpha-2-glycoprotein 1, zinc [Homo sapiens] |
| gil115387099 | brain-specific angiogenesis inhibitor 2 [Homo sapiens] |
| gil178557739 | complement component 4B preproprotein [Homo sapiens] |
| gil8393009 | chromosome 11 open reading frame2 [Homo sapiens] |
| gil40804468 | calcium channel, voltage-dependent, beta 1 subunit isoform 1 [Homo sapiens] |
| gil4502549 | calmodulin 2 [Homo sapiens] |
| gil4502565 | calpain, small subunit 1 [Homo sapiens] |
| gil4502679 | CD63 antigen isoform A [Homo sapiens] |
| gil10835071 | CD74 antigen isoform b [Homo sapiens] |
| gil119627240 | cyclin-dependent kinase inhibitor 2C (p18, inhibits CDK4), isoform CRA_b [Homo sapiens] |
| gil56786147 | cysteine dioxygenase, type I [Homo sapiens] |
| gil169636439 | cholesteryl ester transfer protein, plasma precursor [Homo sapiens] |
| gil5031635 | cofilin 1 (non-muscle) [Homo sapiens] |
| gil4502805 | chromogranin A precursor [Homo sapiens] |
| gil6978649 | choline kinase beta isoform a [Homo sapiens] |
| gil21536286 | brain creatine kinase [Homo sapiens] |
| gil4502847 | cold inducible RNA binding protein [Homo sapiens] |
| gil14917109 | adaptor-related protein complex 2, mu 1 subunit isoform a [Homo sapiens] |
| gil4557471 | adaptor-related protein complex 1, sigma 1 subunit [Homo sapiens] |
| gil41872374 | polo-like kinase 3 [Homo sapiens] |
| gil4502981 | cytochrome c oxidase subunit IV isoform 1 precursor [Homo sapiens] |
| gil4503049 | cysteine-rich protein 2 [Homo sapiens] |
| gil4503051 | collapsin response mediator protein 1 isoform 2 [Homo sapiens] |
| gil4503065 | crystallin, mu isoform 1 [Homo sapiens] |
| gil4503101 | cysteine and glycine-rich protein 2 [Homo sapiens] |
| gil4503107 | cystatin C precursor [Homo sapiens] |
| gil4503139 | cathepsin B preproprotein [Homo sapiens] |
| gil4503211 | cytochrome P450, family 27, subfamily A, polypeptide 1 precursor [Homo sapiens] |
| gil18426915 | drebrin 1 isoform a [Homo sapiens] |
| gil62530384 | dodecenoyl-Coenzyme A delta isomerase precursor [Homo sapiens] |
| gil13259510 | dynactin 1 isoform 1 [Homo sapiens] |
| gil4758138 | DEAD (Asp-Glu-Ala-Asp) box polypeptide 5 [Homo sapiens] |
| gil193783649 | unnamed protein product [Homo sapiens] |
| gil42544174 | deoxythymidylate kinase (thymidylate kinase) [Homo sapiens] |
| gil4503471 | eukaryotic translation elongation factor 1 alpha 1 [Homo sapiens] |
| gil4503475 | eukaryotic translation elongation factor 1 alpha 2 [Homo sapiens] |
| gil4503477 | eukaryotic translation elongation factor 1 beta 2 [Homo sapiens] |
| gil4503481 | eukaryotic translation elongation factor 1 gamma [Homo sapiens] |
| gil4503483 | eukaryotic translation elongation factor 2 [Homo sapiens] |
| gil4826708 | ephrin A3 [Homo sapiens] |
| gil110347457 | EGF-like-domain, multiple 3 [Homo sapiens] |
| gil4503529 | eukaryotic translation initiation factor 4A isoform 1 [Homo sapiens] |
| gil49355765 | ELAV-like protein 3 isoform 2 [Homo sapiens] |
| gil4503571 | enolase 1 [Homo sapiens] |
| gil4758272 | endosulfine alpha isoform 3 [Homo sapiens] |
| gil166362737 | erythrocyte membrane protein band 4.2 isoform 2 [Homo sapiens] |
| gil194097332 | ephrin receptor EphB6 precursor [Homo sapiens] |
| gil4503599 | excision repair cross-complementing 1 isofrom 2 [Homo sapiens] |
| gil46370066 | exostosin 1 [Homo sapiens] |
| gil27886588 | PTK2B protein tyrosine kinase 2 beta isoform b [Homo sapiens] |
| gil41872631 | fatty acid synthase [Homo sapiens] |
| gil4503659 | ubiquitin-like protein fubi and ribosomal protein S30 precursor [Homo sapiens] |
| gil67089147 | farnesyl-diphosphate farnesyltransferase 1 [Homo sapiens] |
| gil209571584 | farnesyl diphosphate synthase isoform b [Homo sapiens] |
| gil4758356 | flap structure-specific endonuclease 1 [Homo sapiens] |
| gil213417614 | fibroblast growth factor 13 isoform 3 [Homo sapiens] |
| gil4503711 | fibroblast growth factor receptor 3 isoform 1 precursor [Homo sapiens] |
| gil4503727 | FK506 binding protein 3, 25kDa [Homo sapiens] |
| gil56682959 | ferritin, heavy polypeptide 1 [Homo sapiens] |
| gil4503817 | 3-ketodihydrosphingosine reductase precursor [Homo sapiens] |
| gil108773793 | glucose-6-phosphate dehydrogenase isoform b [Homo sapiens] |
| gil11497612 | gamma-aminobutyric acid (GABA) B receptor 1 isoform b precursor [Homo sapiens] |
| gil66346737 | glucosidase, alpha; neutral C [Homo sapiens] |
| gil4503911 | growth associated protein 43 isoform 2 [Homo sapiens] |
| gil7669492 | glyceraldehyde-3-phosphate dehydrogenase [Homo sapiens] |
| gil4503971 | GDP dissociation inhibitor 1 [Homo sapiens] |
| gil117938765 | GNAS complex locus isoform g [Homo sapiens] |
| gil33695088 | glycerol-3-phosphate dehydrogenase 1 (soluble) [Homo sapiens] |
| gil47078240 | G protein pathway suppressor 1 isoform 2 [Homo sapiens] |
| gil41406084 | glutathione peroxidase 1 isoform 1 [Homo sapiens] |
| gil75709200 | glutathione peroxidase 4 isoform A precursor [Homo sapiens] |
| gil4504111 | growth factor receptor-bound protein 2 isoform 1 [Homo sapiens] |
| gil29029597 | glutamate receptor KA2 precursor [Homo sapiens] |
| gil57165373 | glutamate receptor, ionotropic, N-methyl D-aspartate-associated protein 1 [Homo sapiens] |
| gil4504183 | glutathione transferase [Homo sapiens] |
| gil4885375 | histone cluster 1, H1c [Homo sapiens] |
| gil4504279 | H3 histone, family 3A [Homo sapiens] |
| gil4504345 | alpha 2 globin [Homo sapiens] |
| gil4885419 | zinc finger and BTB domain containing 48 [Homo sapiens] |
| gil119630055 | high-mobility group nucleosome binding domain 1, isoform CRA_a [Homo sapiens] |
| gil14043070 | heterogeneous nuclear ribonucleoprotein A1 isoform b [Homo sapiens] |
| gil51477708 | heterogeneous nuclear ribonucleoprotein D isoform d [Homo sapiens] |
| gil4826760 | heterogeneous nuclear ribonucleoprotein F [Homo sapiens] |
| gil154759421 | HMT1 hnRNP methyltransferase-like 2 isoform 1 [Homo sapiens] |
| gil4504511 | DnaJ (Hsp40) homolog, subfamily A, member 1 [Homo sapiens] |
| gil5729877 | heat shock 70kDa protein 8 isoform 1 [Homo sapiens] |
| gil154146191 | heat shock protein 90kDa alpha (cytosolic), class A member 1 isoform 2 [Homo sapiens] |
| gil31542947 | chaperonin [Homo sapiens] |
| gil12545395 | islet cell autoantigen 1 [Homo sapiens] |
| gil28178832 | isocitrate dehydrogenase 2 (NADP+), mitochondrial precursor [Homo sapiens] |
| gil28178821 | isocitrate dehydrogenase 3, beta subunit isoform a precursor [Homo sapiens] |
| gil4504619 | insulin-like growth factor binding protein 7 [Homo sapiens] |
| gil125988409 | RED protein [Homo sapiens] |
| gil66933016 | inosine monophosphate dehydrogenase 2 [Homo sapiens] |
| gil68800350 | interleukin-1 receptor-associated kinase 1 isoform 3 [Homo sapiens] |
| gil193785653 | unnamed protein product [Homo sapiens] |
| gil15626999 | inosine triphosphatase isoform a [Homo sapiens] |
| gil4504811 | junction plakoglobin [Homo sapiens] |
| gil20070166 | potassium voltage-gated channel, subfamily F, member 1 [Homo sapiens] |
| gil4758648 | kinesin family member 5B [Homo sapiens] |
| gil116829964 | KiSS-1 metastasis-suppressor [Homo sapiens] |
| gil194306559 | kinesin light chain 1 isoform 3 [Homo sapiens] |
| gil9845502 | ribosomal protein SA [Homo sapiens] |
| gil5031851 | stathmin 1 [Homo sapiens] |
| gil4557032 | L-lactate dehydrogenase B [Homo sapiens] |
| gil5031877 | lamin B1 [Homo sapiens] |
| gil167555125 | low density lipoprotein receptor-related protein 3 [Homo sapiens] |
| gil153945728 | microtubule-associated protein 1B [Homo sapiens] |
| gil8400715 | microtubule-associated protein tau isoform 4 [Homo sapiens] |
| gil14043022 | methionyl-tRNA synthetase [Homo sapiens] |
| gil110347461 | MYC-associated zinc finger protein isoform 1 [Homo sapiens] |
| gil6631095 | minichromosome maintenance complex component 3 [Homo sapiens] |
| gil23510448 | minichromosome maintenance complex component 5 [Homo sapiens] |
| gil5031905 | MyoD family inhibitor [Homo sapiens] |
| gil84311247 | mutant truncated midkine B [Homo sapiens] |
| gil57222568 | myeloid/lymphoid or mixed-lineage leukemia (trithorax homolog, Drosophila); translocated to, 6 [Homo sapiens] |
| gil61835204 | mercaptopyruvate sulfurtransferase isoform 2 [Homo sapiens] |
| gil4505289 | diphosphomevalonate decarboxylase [Homo sapiens] |
| gil17986258 | myosin, light chain 6, alkali, smooth muscle and non-muscle isoform 1 [Homo sapiens] |
| gil154354979 | myosin X [Homo sapiens] |
| gil5174607 | NGFI-A binding protein 2 [Homo sapiens] |
| gil5031931 | nascent polypeptide-associated complex alpha subunit isoform b [Homo sapiens] |
| gil157412270 | heterogeneous nuclear ribonucleoprotein M isoform b [Homo sapiens] |
| gil4557789 | norrin [Homo sapiens] |
| gil4758768 | NADH dehydrogenase (ubiquinone) 1 alpha subcomplex, 10, 42kDa precursor [Homo sapiens] |
| gil4758774 | NADH dehydrogenase (ubiquinone) 1 beta subcomplex, 10, 22kDa [Homo sapiens] |
| gil20149568 | NADH dehydrogenase (ubiquinone) flavoprotein 1, 51kDa precursor [Homo sapiens] |
| gil98986461 | neurogenic differentiation 2 [Homo sapiens] |
| gil187608777 | NF-kappa-B inhibitor-like protein 2 [Homo sapiens] |
| gil119596463 | neuronatin, isoform CRA_a [Homo sapiens] |
| gil4505409 | non-metastatic cells 2, protein (NM23B) expressed in [Homo sapiens] |
| gil10835063 | nucleophosmin 1 isoform 1 [Homo sapiens] |
| gil34098946 | nuclease sensitive element binding protein 1 [Homo sapiens] |
| gil71361682 | nuclear mitotic apparatus protein 1 [Homo sapiens] |
| gil9845505 | ornithine decarboxylase antizyme 1 [Homo sapiens] |
| gil38372882 | RecName: Full=Mitochondrial inner membrane protein OXA1L; AltName: Full=Oxidase assembly 1-like protein; Short=OXA1-like protein; AltName: Full=OXA1Hs; AltName: Full=Hsa; Flags: Precursor |
| gil20070125 | prolyl 4-hydroxylase, beta subunit precursor [Homo sapiens] |
| gil124494254 | ErbB3-binding protein 1 [Homo sapiens] |
| gil4505621 | prostatic binding protein [Homo sapiens] |
| gil4505591 | peroxiredoxin 1 [Homo sapiens] |
| gil93141029 | paralemmin isoform 2 [Homo sapiens] |
| gil106049292 | pyruvate carboxylase precursor [Homo sapiens] |
| gil134019487 | chromatin modifying protein 1A isoform 1 [Homo sapiens] |
| gil4505685 | pyruvate dehydrogenase (lipoamide) alpha 1 precursor [Homo sapiens] |
| gil22202633 | prefoldin subunit 5 isoform alpha [Homo sapiens] |
| gil11321601 | phosphofructokinase, platelet [Homo sapiens] |
| gil16753215 | profilin 2 isoform a [Homo sapiens] |
| gil4505753 | phosphoglycerate mutase 1 (brain) [Homo sapiens] |
| gil20149543 | placental growth factor, vascular endothelial growth factor-related protein [Homo sapiens] |
| gil5453898 | protein (peptidyl-prolyl cis/trans isomerase) NIMA-interacting 1 [Homo sapiens] |
| gil205360954 | polycystin 1 isoform 1 precursor [Homo sapiens] |
| gil33286420 | pyruvate kinase, muscle isoform M1 [Homo sapiens] |
| gil33598948 | phospholipase C gamma 1 isoform a [Homo sapiens] |
| gil9945439 | septin 5 [Homo sapiens] |
| gil156616275 | polymerase (DNA directed), delta 1, catalytic subunit 125kDa [Homo sapiens] |
| gil110618253 | mitochondrial DNA-directed RNA polymerase precursor [Homo sapiens] |
| gil127139033 | cytochrome P450 reductase [Homo sapiens] |
| gil10863927 | peptidylprolyl isomerase A [Homo sapiens] |
| gil4758950 | peptidylprolyl isomerase B precursor [Homo sapiens] |
| gil4826932 | peptidylprolyl isomerase D [Homo sapiens] |
| gil4506003 | protein phosphatase 1, catalytic subunit, alpha isoform 1 [Homo sapiens] |
| gil21361399 | alpha isoform of regulatory subunit A, protein phosphatase 2 [Homo sapiens] |
| gil5453958 | protein phosphatase 5, catalytic subunit [Homo sapiens] |
| gil38257139 | protein kinase, cAMP-dependent, regulatory, type I, beta [Homo sapiens] |
| gil20128774 | mitogen-activated protein kinase 11 [Homo sapiens] |
| gil11386147 | prosaposin isoform a preproprotein [Homo sapiens] |
| gil4506193 | proteasome beta 1 subunit [Homo sapiens] |
| gil25777602 | proteasome 26S non-ATPase subunit 2 [Homo sapiens] |
| gil18543329 | proteasome 26S non-ATPase subunit 9 [Homo sapiens] |
| gil4506217 | proteasome 26S non-ATPase subunit 10 isoform 1 [Homo sapiens] |
| gil46249388 | phosphoserine phosphatase [Homo sapiens] |
| gil28558998 | polypyrimidine tract-binding protein 1 isoform d [Homo sapiens] |
| gil4506281 | pleiotrophin [Homo sapiens] |
| gil4506367 | RAB3A, member RAS oncogene family [Homo sapiens] |
| gil9845511 | ras-related C3 botulinum toxin substrate 1 isoform Rac1 [Homo sapiens] |
| gil4506401 | v-raf-1 murine leukemia viral oncogene homolog 1 [Homo sapiens] |
| gil5453555 | ras-related nuclear protein [Homo sapiens] |
| gil93277122 | RNA binding motif protein 4 [Homo sapiens] |
| gil4506457 | reticulocalbin 2, EF-hand calcium binding domain [Homo sapiens] |
| gil5454004 | D4, zinc and double PHD fingers family 2 [Homo sapiens] |
| gil168983744 | tripartite motif-containing 27 [Homo sapiens] |
| gil156416009 | regulator of G-protein signalling 16 [Homo sapiens] |
| gil4506649 | ribosomal protein L3 isoform a [Homo sapiens] |
| gil16579885 | ribosomal protein L4 [Homo sapiens] |
| gil14591909 | ribosomal protein L5 [Homo sapiens] |
| gil16753227 | ribosomal protein L6 [Homo sapiens] |
| gil15431301 | ribosomal protein L7 [Homo sapiens] |
| gil4506661 | ribosomal protein L7a [Homo sapiens] |
| gil4506663 | ribosomal protein L8 [Homo sapiens] |
| gil15431303 | ribosomal protein L9 [Homo sapiens] |
| gil249371 | laminin receptor homolog [Homo sapiens] |
| gil15431290 | ribosomal protein L11 [Homo sapiens] |
| gil4506597 | ribosomal protein L12 [Homo sapiens] |
| gil15431295 | ribosomal protein L13 [Homo sapiens] |
| gil15431293 | ribosomal protein L15 [Homo sapiens] |
| gil4506617 | ribosomal protein L17 [Homo sapiens] |
| gil4506607 | ribosomal protein L18 [Homo sapiens] |
| gil11415026 | ribosomal protein L18a [Homo sapiens] |
| gil18104948 | ribosomal protein L21 [Homo sapiens] |
| gil4506613 | ribosomal protein L22 proprotein [Homo sapiens] |
| gil4506621 | ribosomal protein L26 [Homo sapiens] |
| gil4506629 | ribosomal protein L29 [Homo sapiens] |
| gil4506633 | ribosomal protein L31 isoform 1 [Homo sapiens] |
| gil16117791 | ribosomal protein L35a [Homo sapiens] |
| gil4506641 | ribosomal protein L37 [Homo sapiens] |
| gil4506643 | ribosomal protein L37a [Homo sapiens] |
| gil4506667 | ribosomal protein P0 [Homo sapiens] |
| gil4506671 | ribosomal protein P2 [Homo sapiens] |
| gil15055539 | ribosomal protein S2 [Homo sapiens] |
| gil15718687 | ribosomal protein S3 [Homo sapiens] |
| gil4506723 | ribosomal protein S3a [Homo sapiens] |
| gil4506725 | ribosomal protein S4, X-linked X isoform [Homo sapiens] |
| gil4506727 | ribosomal protein S4, Y-linked 1 Y isoform [Homo sapiens] |
| gil13904870 | ribosomal protein S5 [Homo sapiens] |
| gil17158044 | ribosomal protein S6 [Homo sapiens] |
| gil4506743 | ribosomal protein S8 [Homo sapiens] |
| gil14141193 | ribosomal protein S9 [Homo sapiens] |
| gil4506679 | ribosomal protein S10 [Homo sapiens] |
| gil14277700 | ribosomal protein S 12 [Homo sapiens] |
| gil4506685 | ribosomal protein S13 [Homo sapiens] |
| gil5032051 | ribosomal protein S 14 [Homo sapiens] |
| gil4506687 | ribosomal protein S15 [Homo sapiens] |
| gil14165469 | ribosomal protein S15a [Homo sapiens] |
| gil4506691 | ribosomal protein S16 [Homo sapiens] |
| gil11968182 | ribosomal protein S18 [Homo sapiens] |
| gil4506701 | ribosomal protein S23 [Homo sapiens] |
| gil214010224 | ribosomal protein S24 isoform f [Homo sapiens] |
| gil4506707 | ribosomal protein S25 [Homo sapiens] |
| gil4506711 | ribosomal protein S27 [Homo sapiens] |
| gil4506713 | ubiquitin and ribosomal protein S27a precursor [Homo sapiens] |
| gil71772583 | ribosomal protein S29 isoform 2 [Homo sapiens] |
| gil45827776 | reticulon 1 isoform C [Homo sapiens] |
| gil16306548 | seryl-tRNA synthetase [Homo sapiens] |
| gil666052 | syndecan-1 [Homo sapiens] |
| gil119577915 | selenoprotein W, 1 [Homo sapiens] |
| gil5902076 | splicing factor, arginine/serine-rich 1 isoform 1 [Homo sapiens] |
| gil72534660 | splicing factor, arginine/serine-rich 7 [Homo sapiens] |
| gil4506913 | sarcoglycan, beta [Homo sapiens] |
| gil21389315 | solute carrier family 25 (mitochondrial carrier; citrate transporter), member 1 precursor [Homo sapiens] |
| gil21071056 | SWI/SNF-related matrix-associated actin-dependent regulator of chromatin a4 isoform B [Homo sapiens] |
| gil55956801 | SWI/SNF related, matrix associated, actin dependent regulator of chromatin, subfamily b, member 1 isoform b [Homo sapiens] |
| gil4507115 | fascin 1 [Homo sapiens] |
| gil29568103 | U1 small nuclear ribonucleoprotein 70 kDa [Homo sapiens] |
| gil4507125 | small nuclear ribonucleoprotein polypeptide B/B' isoform B [Homo sapiens] |
| gil4507127 | small nuclear ribonucleoprotein polypeptide C [Homo sapiens] |
| gil4507171 | secreted protein, acidic, cysteine-rich [Homo sapiens] |
| gil5902122 | spectrin, beta, non-erythrocytic 2 [Homo sapiens] |
| gil38679884 | sorcin isoform b [Homo sapiens] |
| gil63253298 | spermidine synthase [Homo sapiens] |
| gil149999611 | signal recognition particle 14kDa (homologous Alu RNA binding protein) [Homo sapiens] |
| gil15208660 | tripartite motif protein 21 [Homo sapiens] |
| gil108796056 | TROVE domain family, member 2 isoform 1 [Homo sapiens] |
| gil4507239 | signal sequence receptor, beta precursor [Homo sapiens] |
| gil94400932 | syntaxin 5 [Homo sapiens] |
| gil29540543 | sulfotransferase family, cytosolic, 1A, phenol-preferring, member 1 isoform b [Homo sapiens] |
| gil92859638 | synaptotagmin V [Homo sapiens] |
| gil4507373 | beta-tubulin cofactor C [Homo sapiens] |
| gil41350333 | beta-tubulin cofactor D [Homo sapiens] |
| gil4507385 | transcription elongation factor A protein 2 isoform a [Homo sapiens] |
| gil57863259 | T-complex protein 1 isoform b [Homo sapiens] |
| gil32189392 | peroxiredoxin 2 isoform a [Homo sapiens] |
| gil4507521 | transketolase isoform 1 [Homo sapiens] |
| gil21265104 | tetraspanin 7 [Homo sapiens] |
| gil4507645 | triosephosphate isomerase 1 [Homo sapiens] |
| gil4507677 | heat shock protein 90kDa beta, member 1 [Homo sapiens] |
| gil58761484 | chaperonin containing TCP1, subunit 3 isoform c [Homo sapiens] |
| gil91208423 | thyroid receptor-interacting protein 6 [Homo sapiens] |
| gil116256350 | tuberous sclerosis 2 isoform 4 [Homo sapiens] |
| gil49640009 | tetratricopeptide repeat domain 3 [Homo sapiens] |
| gil4507729 | tubulin, beta 2 [Homo sapiens] |
| gil5803207 | U2 small nuclear RNA auxillary factor 1 isoform a [Homo sapiens] |
| gil67191208 | ubiquitin C [Homo sapiens] |
| gil21361091 | ubiquitin carboxyl-terminal esterase L1 [Homo sapiens] |
| gil46593007 | ubiquinol-cytochrome c reductase core protein I [Homo sapiens] |
| gil46877105 | upstream stimulatory factor 2 isoform 2 [Homo sapiens] |
| gil4507877 | vinculin isoform VCL [Homo sapiens] |
| gil6005942 | valosin-containing protein [Homo sapiens] |
| gil4507879 | voltage-dependent anion channel 1 [Homo sapiens] |
| gil62414289 | vimentin [Homo sapiens] |
| gil88853069 | vitronectin precursor [Homo sapiens] |
| gil190684675 | X-ray repair cross complementing protein 1 [Homo sapiens] |
| gil4507949 | tyrosine 3-monooxygenase/tryptophan 5-monooxygenase activation protein, beta polypeptide [Homo sapiens] |
| gil5803225 | tyrosine 3/tryptophan 5 -monooxygenase activation protein, epsilon polypeptide [Homo sapiens] |
| gil4507951 | tyrosine 3-monooxygenase/tryptophan 5-monooxygenase activation protein, eta polypeptide [Homo sapiens] |
| gil4507961 | zinc finger protein 36, C3H type, homolog [Homo sapiens] |
| gil4827071 | zinc finger protein 9 isoform 3 [Homo sapiens] |
| gil23510455 | zinc finger protein 41 [Homo sapiens] |
| gil27545332 | zinc finger protein 133 [Homo sapiens] |
| gil145977222 | zinc finger protein 154 [Homo sapiens] |
| gil32698678 | zinc finger protein 213 [Homo sapiens] |
| gil17986283 | tubulin, alpha 1a [Homo sapiens] |
| gil197333755 | interferon-related developmental regulator 2 [Homo sapiens] |
| gil54607089 | semaphorin 3B isoform 2 precursor [Homo sapiens] |
| gil4758112 | HLA-B associated transcript 1 [Homo sapiens] |
| gil4502483 | Ras association (RalGDS/AF-6) domain family (N-terminal) member 7 [Homo sapiens] |
| gil12962937 | achalasia, adrenocortical insufficiency, alacrimia (Allgrove, triple-A) [Homo sapiens] |
| gil134142817 | es1 protein isoform Ib precursor [Homo sapiens] |
| gil4507691 | transformation/transcription domain-associated protein [Homo sapiens] |
| gil4507183 | speckle-type POZ protein [Homo sapiens] |
| gil7657134 | glyceronephosphate O-acyltransferase [Homo sapiens] |
| gil32307161 | cullin 1 [Homo sapiens] |
| gil41872527 | diacylglycerol kinase zeta isoform 3 [Homo sapiens] |
| gil32189362 | PTPRF interacting protein alpha 3 [Homo sapiens] |
| gil55769587 | probable nucleolar complex protein 14 [Homo sapiens] |
| gil117553627 | acetylserotonin O-methyltransferase-like [Homo sapiens] |
| gil189571687 | nuclear autoantigen of 14 kDa [Homo sapiens] |
| gil16933557 | dachsous 1 precursor [Homo sapiens] |
| gil4505813 | dynein light chain 1 [Homo sapiens] |
| gil4503525 | eukaryotic translation initiation factor 3, subunit C [Homo sapiens] |
| gil4503523 | eukaryotic translation initiation factor 3 subunit D [Homo sapiens] |
| gil4503515 | eukaryotic translation initiation factor 3, subunit 3 gamma, 40kDa [Homo sapiens] |
| gil4507285 | syntaxin 10 [Homo sapiens] |
| gil4505705 | phosphoprotein enriched in astrocytes 15 [Homo sapiens] |
| gil4506903 | splicing factor, arginine/serine-rich 9 [Homo sapiens] |
| gil4504079 | glycosylphosphatidylinositol anchor attachment protein 1 [Homo sapiens] |
| gil46909598 | a disintegrin and metalloproteinase domain 15 isoform 5 preproprotein [Homo sapiens] |
| gil4505229 | Fas-associated via death domain [Homo sapiens] |
| gil47933379 | N-ethylmaleimide-sensitive factor attachment protein, alpha [Homo sapiens] |
| gil19923191 | minichromosome maintenance complex component 3 associated protein [Homo sapiens] |
| gil14210504 | adaptor-related protein complex 1, mu 1 subunit isoform 2 [Homo sapiens] |
| gil4507913 | Wiskott-Aldrich syndrome protein family member 1 [Homo sapiens] |
| gil48762942 | huntingtin interacting protein-1-related [Homo sapiens] |
| gil78000181 | ribosomal protein L14 [Homo sapiens] |
| gil11141861 | claudin 9 [Homo sapiens] |
| gil115527080 | metastasis associated protein [Homo sapiens] |
| gil4759276 | RNA, U3 small nucleolar interacting protein 2 [Homo sapiens] |
| gil190358517 | RAB11B, member RAS oncogene family [Homo sapiens] |
| GI:284005309 | ATP-dependent DNA helicase Q4 [Homo sapiens] |
| gil3834621 | homer-3 [Homo sapiens] |
| gil10834682 | PP3958 [Homo sapiens] |
| gil33695117 | p53-induced protein [Homo sapiens] |
| gil18344454 | sperm associated antigen 7 [Homo sapiens] |
| gil4758496 | H2A histone family, member Y isoform 2 [Homo sapiens] |
| gil195927015 | phosphatidylinositol transfer protein, membrane-associated isoform a [Homo sapiens] |
| gil116256445 | nuclear receptor co-repressor 2 isoform 2 [Homo sapiens] |
| gil22027622 | TNF receptor-associated factor 4 [Homo sapiens] |
| gil57242774 | hypothetical protein LOC9703 [Homo sapiens] |
| gil7661916 | lysosomal protein transmembrane 4 alpha [Homo sapiens] |
| gil7661890 | sorting nexin 17 [Homo sapiens] |
| gil17999539 | DEAH (Asp-Glu-Ala-His) box polypeptide 38 [Homo sapiens] |
| gil55769518 | inositol hexakisphosphate kinase 1 isoform 2 [Homo sapiens] |
| gil110624774 | mannose receptor, C type 2 [Homo sapiens] |
| gil55925608 | kelch-like 21 [Homo sapiens] |
| gil151108509 | Rho GTPase-activating protein RICH2 [Homo sapiens] |
| gil55770886 | ubiquitin specific protease 3 [Homo sapiens] |
| gil14110407 | heterogeneous nuclear ribonucleoprotein D-like [Homo sapiens] |
| gil62750347 | histone deacetylase 5 isoform 1 [Homo sapiens] |
| gil194294519 | nuclear receptor subfamily 1, group H, member 3 isoform c [Homo sapiens] |
| gil65301139 | ATPase, class II, type 9A [Homo sapiens] |
| gil5031595 | actin related protein 2/3 complex subunit 4 isoform a [Homo sapiens] |
| gil6912540 | nucleotide binding protein 2 (MinD homolog, E. coli) [Homo sapiens] |
| gil5031569 | ARP1 actin-related protein 1 homolog A, centractin alpha [Homo sapiens] |
| gil155722983 | TNF receptor-associated protein 1 [Homo sapiens] |
| gil121114294 | RAS p21 protein activator 4 isoform 1 [Homo sapiens] |
| gil5031633 | FERM, RhoGEF, and pleckstrin domain protein 1 isoform 1 [Homo sapiens] |
| gil5032031 | RNA binding motif protein 5 [Homo sapiens] |
| gil5032133 | eukaryotic translation initiation factor 1 [Homo sapiens] |
| gil74735668 | RecName: Full=Major facilitator superfamily domain-containing protein 10; AltName: Full=Tetracycline transporter-like protein |
| gil5031669 | cyclin-dependent kinase 2 associated protein 2 [Homo sapiens] |
| gil56181387 | STIP1 homology and U-box containing protein 1 [Homo sapiens] |
| gil95147542 | amyloid beta precursor protein-binding, family B, member 3 isoform a [Homo sapiens] |
| gil116256481 | TNFAIP3 interacting protein 1 [Homo sapiens] |
| gil57013276 | tubulin, alpha, ubiquitous [Homo sapiens] |
| gil50592996 | tubulin, beta, 4 [Homo sapiens] |
| gil21361322 | tubulin, beta 4 [Homo sapiens] |
| gil5174735 | tubulin, beta, 2 [Homo sapiens] |
| gil37655183 | N-myc downstream regulated 1 [Homo sapiens] |
| gil5174447 | guanine nucleotide binding protein (G protein), beta polypeptide 2-like 1 [Homo sapiens] |
| gil5454064 | RNA binding motif protein 14 [Homo sapiens] |
| gil17136143 | olfactomedin related ER localized protein isoform 1 [Homo sapiens] |
| gil29893564 | microspherule protein 1 isoform 1 [Homo sapiens] |
| gil5174723 | translocase of outer mitochondrial membrane 40 [Homo sapiens] |
| gil9257197 | BAI1-associated protein 2 isoform 1 [Homo sapiens] |
| gil19923354 | zinc finger protein 238 isoform 2 [Homo sapiens] |
| gil23397429 | eukaryotic translation initiation factor 3, subunit M [Homo sapiens] |
| gil5453595 | adenylyl cyclase-associated protein [Homo sapiens] |
| gil5454166 | vesicle transport through interaction with t-SNAREs 1B [Homo sapiens] |
| gil18379349 | vesicle amine transport protein 1 [Homo sapiens] |
| gil6102858 | hypothetical protein [Homo sapiens] |
| gil36287060 | K(lysine) acetyltransferase 5 isoform 3 [Homo sapiens] |
| gil5453838 | Sjogren syndrome/scleroderma autoantigen 1 [Homo sapiens] |
| gil95113651 | glutaredoxin 3 [Homo sapiens] |
| gil5453629 | dynactin 2 [Homo sapiens] |
| gil26051229 | mitochondrial ribosomal protein L28 [Homo sapiens] |
| gil58331185 | chaperonin containing TCP1, subunit 7 isoform b [Homo sapiens] |
| gill97383077 | PDZ and LIM domain 5 isoform a [Homo sapiens] |
| gil32454737 | tripartite motif-containing 3 [Homo sapiens] |
| gill71184421 | thioredoxin interacting protein [Homo sapiens] |
| gil5454030 | RAS-related on chromosome 22 isoform a [Homo sapiens] |
| gil5729794 | CUG triplet repeat, RNA-binding protein 1 isoform 1 [Homo sapiens] |
| gil10835246 | Kruppel-like factor 1 (erythroid) [Homo sapiens] |
| gil5729999 | Ras-related GTP binding A [Homo sapiens] |
| gil5729953 | nuclear distribution gene C homolog [Homo sapiens] |
| gil38261971 | cyclic AMP-regulated phosphoprotein, 21 kD isoform 1 [Homo sapiens] |
| gil28269672 | serologically defined colon cancer antigen 8 [Homo sapiens] |
| gil42544159 | heat shock 105kD [Homo sapiens] |
| gil5729875 | progesterone receptor membrane component 1 [Homo sapiens] |
| gil5730015 | RUN domain containing 3A [Homo sapiens] |
| gil19387846 | melanoma antigen family D, 2 [Homo sapiens] |
| gil5802970 | AFG3 ATPase family gene 3-like 2 [Homo sapiens] |
| gil5803023 | lectin, mannose-binding 2 precursor [Homo sapiens] |
| gil5803013 | endoplasmic reticulum protein 29 isoform 1 precursor [Homo sapiens] |
| gil5870893 | solute carrier family 38, member 3 [Homo sapiens] |
| gil166795250 | kinesin family member 2C [Homo sapiens] |
| gil33286446 | opioid growth factor receptor [Homo sapiens] |
| gil34850061 | superiorcervical ganglia, neural specific 10 [Homo sapiens] |
| gil16554627 | WD repeat domain 5 [Homo sapiens] |
| gil66472382 | nischarin [Homo sapiens] |
| gil6005860 | ribosomal protein L35 [Homo sapiens] |
| gil19966764 | lysophospholipase II [Homo sapiens] |
| gil45446743 | DEAD box polypeptide 42 protein [Homo sapiens] |
| gil32528286 | acyl-CoA thioesterase 7 isoform hBACHd [Homo sapiens] |
| gil6005764 | GABA(A) receptor-associated protein [Homo sapiens] |
| gil11559929 | coatomer protein complex, subunit gamma 1 [Homo sapiens] |
| gil17978512 | poly-U binding splicing factor 60KDa isoform a [Homo sapiens] |
| gil41281557 | latrophilin 1 isoform 2 precursor [Homo sapiens] |
| gil41349441 | SEC31 homolog A isoform 2 [Homo sapiens] |
| gil57242755 | calsyntenin 1 isoform 2 [Homo sapiens] |
| gil20336290 | DEAH (Asp-Glu-Ala-His) box polypeptide 30 isoform 2 [Homo sapiens] |
| gil21396480 | RNA binding protein (autoantigenic, hnRNP-associated with lethal yellow) short isoform [Homo sapiens] |
| gil66932902 | SREBF chaperone protein [Homo sapiens] |
| gil58761546 | amine oxidase (flavin containing) domain 2 isoform b [Homo sapiens] |
| gil42516565 | ubiquitin specific protease 33 isoform 2 [Homo sapiens] |
| gil83716024 | kinesin family member 21B [Homo sapiens] |
| gil24307983 | FtsJ methyltransferase domain containing 2 [Homo sapiens] |
| gil122937251 | jumonji domain containing 3, histone lysine demethylase [Homo sapiens] |
| gil155722994 | zinc finger CCCH-type containing 3 [Homo sapiens] |
| gil67763814 | death-inducing-protein [Homo sapiens] |
| gil112421108 | capicua homolog [Homo sapiens] |
| gil154689719 | hypothetical protein LOC23231 [Homo sapiens] |
| gil21327667 | block of proliferation 1 [Homo sapiens] |
| gil62241044 | exocyst complex component 7 isoform b [Homo sapiens] |
| gil82659109 | retinoblastoma-associated factor 600 [Homo sapiens] |
| gil21624607 | coactosin-like 1 [Homo sapiens] |
| gil18034692 | CDW92 antigen [Homo sapiens] |
| gil7657455 | pescadillo homolog 1, containing BRCT domain [Homo sapiens] |
| gil45827731 | scribble isoform a [Homo sapiens] |
| gil6912458 | calcineurin binding protein 1 [Homo sapiens] |
| gil6912674 | SNAP-associated protein [Homo sapiens] |
| gil7524354 | dimethylarginine dimethylaminohydrolase 2 [Homo sapiens] |
| gil164519084 | RAB GTPase activating protein 1 [Homo sapiens] |
| gil112363070 | hsp70-interacting protein [Homo sapiens] |
| gil72534684 | phospholipase D family, member 3 [Homo sapiens] |
| gil6912490 | transcription factor MAFF [Homo sapiens] |
| gil52630440 | FK506-binding protein 8 [Homo sapiens] |
| gil7657345 | mitochondrial carrier homolog 1 [Homo sapiens] |
| gil169790811 | SHC (Src homology 2 domain containing) transforming protein 2 [Homo sapiens] |
| gil6912586 | 6-phosphogluconolactonase [Homo sapiens] |
| gil6912238 | peroxiredoxin 5 isoform a precursor [Homo sapiens] |
| gil4507975 | zinc finger protein 345 [Homo sapiens] |
| gil32698704 | hypothetical protein LOC25851 [Homo sapiens] |
| gil17530785 | breast cancer metastasis suppressor 1 isoform 1 [Homo sapiens] |
| gil36796743 | methylenetetrahydrofolate dehydrogenase (NADP+ dependent) 1-like [Homo sapiens] |
| gil20070260 | cofactor of BRCA1 [Homo sapiens] |
| gil195972913 | nasal embryonic LHRH factor isoform d [Homo sapiens] |
| gil7661696 | hypothetical protein LOC26017 [Homo sapiens] |
| gil5911953 | hypothetical protein [Homo sapiens] |
| gi141872443 | TRPC4-associated protein isoform b [Homo sapiens] |
| gil31542521 | N-acetyltransferase 9 (GCN5-related, putative) [Homo sapiens] |
| gil150170699 | kinesin family member 26A [Homo sapiens] |
| gil28416431 | GTPase, IMAP family member 2 [Homo sapiens] |
| gil215277017 | protein tyrosine phosphatase, non-receptor type 18 isoform 2 [Homo sapiens] |
| gi146367787 | poly(A) binding protein, cytoplasmic 1 [Homo sapiens] |
| gil7656952 | calcyclin binding protein isoform 1 [Homo sapiens] |
| gil7657599 | sorting nexin 5 [Homo sapiens] |
| gil148727368 | bromodomain and PHD finger containing, 3 [Homo sapiens] |
| gil7657514 | GTP-binding protein RH06 [Homo sapiens] |
| gil140560994 | growth inhibition and differentiation related protein 86 [Homo sapiens] |
| gil166235178 | PDZ and LIM domain protein 3 isoform b [Homo sapiens] |
| gil7657196 | zinc finger protein 330 [Homo sapiens] |
| gil7657381 | PRP19/PSO4 pre-mRNA processing factor 19 homolog [Homo sapiens] |
| gil7019519 | proprotein convertase subtilisin/kexin type 1 inhibitor precursor [Homo sapiens] |
| gil7661734 | mRNA decapping enzyme [Homo sapiens] |
| gil7661744 | basic leucine zipper and W2 domains 2 [Homo sapiens] |
| gil27414497 | zinc finger CCCH-type containing 7A [Homo sapiens] |
| gil40548422 | chromatin modifying protein 4A [Homo sapiens] |
| gil8392875 | transcription factor IIB [Homo sapiens] |
| gil58761502 | GTP-binding protein PTD004 isoform 2 [Homo sapiens] |
| gil54607124 | RNA binding motif protein 15B [Homo sapiens] |
| gil194248095 | epsin 1 isoform b [Homo sapiens] |
| gil8574093 | GLTSCR2, glioma tumor suppressor candidate region protein 2 (AF182076_1) [Homo sapiens] |
| gil7305503 | stomatin (EPB72)-like 2 [Homo sapiens] |
| gil7657465 | podocalyxin-like 2 [Homo sapiens] |
| gil14670375 | SCG10-like-protein [Homo sapiens] |
| gil7705716 | nitric oxide synthase interacting protein [Homo sapiens] |
| gil56676379 | intraflagellar transport 52 homolog [Homo sapiens] |
| gil166795285 | coenzyme Q4 homolog [Homo sapiens] |
| gil7705851 | coiled-coil-helix-coiled-coil-helix domain containing 2 [Homo sapiens] |
| gil189083821 | inhibitor of growth family, member 4 isoform 9 [Homo sapiens] |
| gil7705889 | EGF-like-domain, multiple 7 [Homo sapiens] |
| gil156142184 | ankyrin repeat domain 39 [Homo sapiens] |
| gil108773808 | hypothetical protein LOC51244 [Homo sapiens] |
| gil75677349 | poly(A) binding protein interacting protein 2 [Homo sapiens] |
| gil75677385 | neugrin, neurite outgrowth associated isoform 2 [Homo sapiens] |
| gil119618613 | processing of precursor 5, ribonuclease P/MRP subunit (S. cerevisiae), isoform CRA_c [Homo sapiens] |
| gil7705433 | eukaryotic translation initiation factor 3 subunit 6 interacting protein [Homo sapiens] |
| gil7705294 | actin-like 6B [Homo sapiens] |
| gil55769520 | inositol hexakisphosphate kinase 2 isoform a [Homo sapiens] |
| gil156416000 | ubiquitin-fold modifier conjugating enzyme 1 [Homo sapiens] |
| gil193788722 | hypothetical protein LOC51507 [Homo sapiens] |
| gil189409150 | chromatin modifying protein 5 [Homo sapiens] |
| gil7705266 | NCK interacting protein with SH3 domain isoform 1 [Homo sapiens] |
| gil18777675 | APC11 anaphase promoting complex subunit 11 isoform 2 [Homo sapiens] |
| gil17706284 | HMP19 protein [Homo sapiens] |
| gil7706359 | RAS, dexamethasone-induced 1 [Homo sapiens] |
| gil7706429 | trafficking protein particle complex 2-like [Homo sapiens] |
| gil49574502 | NAD(P)H:quinone oxidoreductase type 3, polypeptide A2 [Homo sapiens] |
| gil134947986 | zinc finger protein 44 [Homo sapiens] |
| gil20336313 | B-cell CLL/lymphoma 11A isoform 3 [Homo sapiens] |
| gil33695090 | prostate tumor overexpressed 1 [Homo sapiens] |
| gil11612655 | FXYD domain-containing ion transport regulator 6 [Homo sapiens] |
| gil82830424 | gon-4-like isoform a [Homo sapiens] |
| gil157388902 | semaphorin 4C [Homo sapiens] |
| gil8923417 | ADP-ribosylhydrolase like 2 [Homo sapiens] |
| gil57863312 | uridine-cytidine kinase 1-like 1 [Homo sapiens] |
| gil194018570 | coiled-coil domain containing 109B [Homo sapiens] |
| gil157388917 | FEZ family zinc finger 2 [Homo sapiens] |
| GI:268832172 | ATP synthase subunit s-like protein isoform 4 [Homo sapiens] |
| gil38146111 | DALR anticodon binding domain containing 3 isoform 2 [Homo sapiens] |
| gil39780571 | misato [Homo sapiens] |
| gil8922794 | U3 snoRNP protein 3 homolog [Homo sapiens] |
| gil37537687 | zinc finger protein 444 [Homo sapiens] |
| gil218751874 | hairy and enhancer of split 6 isoform b [Homo sapiens] |
| gil8923920 | H2A histone family, member Y2 [Homo sapiens] |
| gil209977067 | OTU domain containing 5 isoform c [Homo sapiens] |
| gil7020625 | unnamed protein product [Homo sapiens] |
| gil209862873 | zinc finger protein 692 isoform 1 [Homo sapiens] |
| gil8922328 | RNA binding motif protein 22 [Homo sapiens] |
| gil140806225 | zinc finger protein 64 isoform b [Homo sapiens] |
| gil17999541 | vacuolar protein sorting 35 [Homo sapiens] |
| gil30410720 | exocyst complex component 1 isoform 1 [Homo sapiens] |
| gil8922867 | hypothetical protein LOC55776 [Homo sapiens] |
| gil209976986 | PCI domain containing 2 [Homo sapiens] |
| gil8923898 | zinc finger protein 313 [Homo sapiens] |
| gil13123776 | DNA methyltransferase 1 associated protein 1 [Homo sapiens] |
| gil9790193 | reprimo, TP53 dependant G2 arrest mediator candidate [Homo sapiens] |
| gil9050060 | NPDC-1 [Homo sapiens] |
| gil11034855 | TMEM9 domain family, member B [Homo sapiens] |
| gil119608262 | SH3-domain GRB2-like endophilin B2, isoform CRA_b [Homo sapiens] |
| gil47174864 | exosome component Rrp46 [Homo sapiens] |
| gil57863279 | Meis1, myeloid ecotropic viral integration site 1 homolog 3 isoform 2 [Homo sapiens] |
| gil94536838 | poly (ADP-ribose) polymerase family, member 6 [Homo sapiens] |
| gil116174742 | family with sequence similarity 20, member C [Homo sapiens] |
| gil9910382 | mitochondrial import receptor Tom22 [Homo sapiens] |
| gil197100773 | arginyl-tRNA synthetase 2, mitochondrial precursor [Homo sapiens] |
| gil23943862 | N-acetyltransferase 14 (GCN5-related, putative) [Homo sapiens] |
| gil140560988 | zinc finger protein SBZF3 [Homo sapiens] |
| gil21704283 | junctophilin 3 [Homo sapiens] |
| gil10190720 | NmrA-like family domain containing 1 [Homo sapiens] |
| gil149274653 | patched domain containing 2 [Homo sapiens] |
| gil33469964 | splicing factor 4 [Homo sapiens] |
| gil145275185 | MYG1 protein [Homo sapiens] |
| gil70778869 | invasion inhibitory protein 45 [Homo sapiens] |
| gil192449449 | ubiquitin-conjugating enzyme E20 [Homo sapiens] |
| gil149158725 | N-terminal EF-hand calcium binding protein 3 isoform 1 [Homo sapiens] |
| gil74048537 | praja 1 isoform b [Homo sapiens] |
| gil84626578 | G protein beta subunit-like [Homo sapiens] |
| gil11968051 | nuclear prelamin A recognition factor-like [Homo sapiens] |
| gil18027756 | unknown [Homo sapiens] |
| gil40255020 | hypothetical protein LOC64769 [Homo sapiens] |
| gil12383064 | fibronectin type III domain containing 4 [Homo sapiens] |
| gil12597653 | 5'-nucleotidase domain containing 2 isoform 2 [Homo sapiens] |
| gil15721937 | mitochondrial ribosomal protein S24 [Homo sapiens] |
| gil16554609 | mitochondrial ribosomal protein S 11 isoform a [Homo sapiens] |
| gil14165272 | PTEN induced putative kinase 1 precursor [Homo sapiens] |
| gil130978962 | adrenocortical dysplasia homolog isoform 2 [Homo sapiens] |
| gil145580615 | KRI1 homolog [Homo sapiens] |
| gil13491174 | MARCKS-like 1 [Homo sapiens] |
| gil32455273 | WNK lysine deficient protein kinase 2 [Homo sapiens] |
| gil38196969 | ZSCAN18 protein [Homo sapiens] |
| gil30581160 | ganglioside-induced differentiation-associated protein 1-like 1 [Homo sapiens] |
| gil13128992 | hypothetical protein MGC2803 [Homo sapiens] |
| gi\|148596996 | CUE domain containing 2 [Homo sapiens] |
| gil113129000 | meteorin, glial cell differentiation regulator [Homo sapiens] |
| gil13129004 | GIY-YIG domain containing 2 isoform 1 [Homo sapiens] |
| gil83776596 | CaM kinase-like vesicle-associated [Homo sapiens] |
| gil13129128 | hypothetical protein LOC79095 [Homo sapiens] |
| gil13236587 | transmembrane protein 43 [Homo sapiens] |
| gil170932471 | hypothetical protein LOC79703 [Homo sapiens] |
| gil146198654 | zinc finger protein 768 [Homo sapiens] |
| gil13376339 | plasticity-related protein 2 [Homo sapiens] |
| gil186910323 | hypothetical protein LOC79969 isoform 2 [Homo sapiens] |
| gil47174859 | tripartite motif-containing 46 [Homo sapiens] |
| gil11640560 | MSTP016 [Homo sapiens] |
| gil41872403 | flavin adenine dinucleotide synthetase isoform 2 [Homo sapiens] |
| gil18027792 | unknown [Homo sapiens] |
| gil13540531 | integrin-linked kinase-associated protein phosphatase 2C [Homo sapiens] |
| gil21915167 | glutamate receptor, ionotropic, N-methyl D-aspartate-like 1A isoform 1 [Homo sapiens] |
| gi\|21361947 | stathmin-like 4 [Homo sapiens] |
| gil13540594 | lectin, mannose-binding 2-like isoform 2 [Homo sapiens] |
| gil18079254 | p53 and DNA damage-regulated protein [Homo sapiens] |
| gil13540614 | coiled-coil domain containing 130 [Homo sapiens] |
| gil88758580 | cyclin L2 isoform A [Homo sapiens] |
| gil13569962 | RAB1B, member RAS oncogene family [Homo sapiens] |
| gil13654276 | queuine tRNA-ribosyltransferase 1 [Homo sapiens] |
| gil194306564 | hypothetical protein LOC81926 isoform 2 [Homo sapiens] |
| gil54607108 | zinc finger, MIZ-type containing 2 isoform 1 [Homo sapiens] |
| gil32880229 | selenoprotein O [Homo sapiens] |
| gil117956405 | yippee-like 3 [Homo sapiens] |
| gil20070354 | histone deacetylase 10 [Homo sapiens] |
| gil187936946 | hypothetical protein LOC84140 [Homo sapiens] |
| gil47716512 | mex-3 homolog B [Homo sapiens] |
| gil148596959 | hypothetical protein LOC84293 [Homo sapiens] |
| gil14150141 | programmed cell death 2-like [Homo sapiens] |
| gi\|21362050 | coiled-coil domain containing 115 [Homo sapiens] |
| gil32129199 | cytokine induced protein 29 kDa [Homo sapiens] |
| gil148806884 | AKT1 substrate 1 (proline-rich) [Homo sapiens] |
| gil31543983 | ADP-ribosylation factor GTPase activating protein 2 [Homo sapiens] |
| gil24308326 | BR serine/threonine kinase 1 [Homo sapiens] |
| gil119624572 | tau tubulin kinase 1, isoform CRA_a [Homo sapiens] |
| gil14249132 | brain expressed X-linked 2 [Homo sapiens] |
| gi\|40548382 | multidrug resistance-related protein [Homo sapiens] |
| gil10438605 | unnamed protein product [Homo sapiens] |
| gil42734379 | THAP domain containing, apoptosis associated protein 3 [Homo sapiens] |
| gil74713747 | RecName: Full=ATP-binding domain-containing protein 3; AltName: Full=Cancer-associated gene protein |
| gi\|89191850 | Yip1 interacting factor homolog B isoform 4 [Homo sapiens] |
| gil111074524 | Src homology 2 domain containing F [Homo sapiens] |
| gil55742736 | zinc finger protein 700 [Homo sapiens] |
| gil53729359 | leucine rich repeat and sterile alpha motif containing 1 [Homo sapiens] |
| gil27552770 | sp1A/ryanodine receptor domain and SOCS box containing 3 [Homo sapiens] |
| gil50086624 | integrator complex subunit 4 [Homo sapiens] |
| gil32455236 | helicase (DNA) B [Homo sapiens] |
| gil217416379 | heterogeneous nuclear ribonucleoprotein L-like isoform 2 [Homo sapiens] |
| gil16445029 | immunoglobulin superfamily, member 8 [Homo sapiens] |
| gil34878735 | F-box protein 44 isoform 1 [Homo sapiens] |
| gil19923935 | coiled-coil domain containing 101 [Homo sapiens] |
| gil156564240 | coiled-coil domain containing 104 [Homo sapiens] |
| gil209571507 | HGFL protein isoform 2 [Homo sapiens] |
| gil112821681 | G protein-regulated inducer of neurite outgrowth 1 [Homo sapiens] |
| gil22035616 | oxysterol-binding protein-like protein 7 [Homo sapiens] |
| gil115511030 | hypothetical protein LOC115004 [Homo sapiens] |
| gi\|30520320 | ring finger protein 166 [Homo sapiens] |
| gil16945972 | kelch domain containing 3 [Homo sapiens] |
| gil28274701 | zinc finger protein 511 [Homo sapiens] |
| gil50557648 | zinc finger, FYVE domain containing 27 isoform c [Homo sapiens] |
| gil21389389 | SLAIN motif family, member 1 B [Homo sapiens] |
| gil160420328 | iron-sulfur cluster assembly 2 [Homo sapiens] |
| gil124517699 | ankyrin repeat domain 13B [Homo sapiens] |
| gil38788219 | zinc finger protein 428 [Homo sapiens] |
| gil20270343 | ADP-ribosylation factor-like 8A [Homo sapiens] |
| gil28827795 | chromatin modifying protein 4B [Homo sapiens] |
| gil50511941 | EMI domain containing 1 [Homo sapiens] |
| GI:285026434 | U8 snoRNA-decapping enzyme isoform 2 [Homo sapiens] |
| gil30520329 | lysophosphatidic acid acyltransferase zeta [Homo sapiens] |
| gil133922582 | zinc finger protein 358 [Homo sapiens] |
| gil17933774 | transcription elongation factor A (SII)-like 2 [Homo sapiens] |
| gil18129632 | sterile alpha motif domain containing 10 [Homo sapiens] |
| gil18152783 | ribosomal protein L10-like protein [Homo sapiens] |
| gil190341093 | fibroblast growth factor binding protein 3 [Homo sapiens] |
| gil58761496 | heterogeneous nuclear ribonucleoprotein Al-like [Homo sapiens] |
| gil119597916 | hCG1820887 [Homo sapiens] |
| gi\|122937289 | kinesin family member 18B [Homo sapiens] |
| gil22748979 | transmembrane protein 199 [Homo sapiens] |
| gil46275820 | tetratricopeptide repeat domain 9B [Homo sapiens] |
| gil119576613 | hCG20425, isoform CRA_a [Homo sapiens] |
| gil22749047 | coiled-coil domain containing 24 [Homo sapiens] |
| gi\|40555765 | RNF187 protein [Homo sapiens] |
| gi\|21389515 | ring finger protein 145 [Homo sapiens] |
| gil63003894 | hypothetical protein LOC154467 [Homo sapiens] |
| gil110681708 | zinc finger protein 579 [Homo sapiens] |
| gil22749235 | zinc finger protein 709 [Homo sapiens] |
| gil24475814 | polymerase (RNA) III (DNA directed) polypeptide H (22.9kD) isoform a [Homo sapiens] |
| gil21699088 | zinc finger protein 627 [Homo sapiens] |
| gil29788785 | tubulin, beta [Homo sapiens] |
| gil67906195 | ankyrin repeat and sterile alpha motif domain containing 6 [Homo sapiens] |
| gil40288203 | hypothetical protein LOC219287 isoform 1 [Homo sapiens] |
| gil32698938 | cornichon homolog 2 [Homo sapiens] |
| gil25014109 | selenoprotein H [Homo sapiens] |
| gil119588217 | hypothetical protein LOC283174 [Homo sapiens] |
| gil32698950 | hypothetical protein LOC283870 [Homo sapiens] |
| gil119592278 | hematopoietic signal peptide-containing, isoform CRA_b [Homo sapiens] |
| gi\|194378374 | unnamed protein product [Homo sapiens] |
| gil87196600 | polyserase 3 [Homo sapiens] |
| gil56090146 | jumonji domain containing 8 [Homo sapiens] |
| gil29788768 | tubulin, beta 2B [Homo sapiens] |
| gil30026034 | solute carrier family 35, member B2 [Homo sapiens] |
| gil126722894 | hypothetical protein LOC348262 [Homo sapiens] |
| gi\|187281616 | NADH-ubiquinone oxidoreductase Fe-S protein 7 precursor [Homo sapiens] |
| gil158256596 | unnamed protein product [Homo sapiens] |
| gil167466201 | WAS protein family homolog 1 [Homo sapiens] |
| gi158219048 | hairy and enhancer of split 5 [Homo sapiens] |
| gil157819143 | hypothetical protein LOC402665 [Homo sapiens] |
| gil39992616 | PIM3 protein [Homo sapiens] |
| gil169161838 | PREDICTED: similar to hCG2040565 [Homo sapiens] |
| gil205830928 | RecName: Full=TLC domain-containing protein 2 |
| gil119964728 | D-2-hydroxyglutarate dehydrogenase precursor [Homo sapiens] |
| gil124249392 | DNL-type zinc finger [Homo sapiens] |

## Patentansprüche

1. Verwendung der Markersequenzen zur Diagnose von Systemischer Lupus erythematodes, wobei mindestens eine Markersequenz einer cDNA ausgewählt aus der Gruppe SEQ 1 - 716 und / oder SEQ 1a-716a oder jeweils ein dafür kodierendes Protein oder jeweils einer Teilsequenz oder Fragment davon an oder von einem zu untersuchenden Patienten bestimmt wird.

2. Verwendung der Markersequenzen zur Diagnose von Systemischer Lupus erythematodes nach Anspruch 1, **dadurch gekennzeichnet**, mindestens 2 bis 5 oder 10, vorzugsweise 30 bis 50 Markersequenzen oder 50 bis 100 oder mehr Markersequenzen an oder von einem zu untersuchenden Patienten bestimmt wird.

3. Verwendung der Markersequenzen zur Diagnose von Systemischer Lupus erythematodes nach Anspruch 1 oder 2, **dadurch gekennzeichnet, dass** die Bestimmung mittels in-vitro Diagnose erfolgt.

4. Verwendung einer Markersequenz einer cDNA jeweils ausgewählt aus der Gruppe SEQ 1 - 716 und / oder SEQ 1a-716a oder jeweils ein dafür kodierendes Protein oder jeweils einer Teilsequenz oder Fragment davon als Diagnostikum.

5. Verwendung der Markersequenzen zur Diagnose von Systemischer Lupus erythematodes nach einem der vorhergehenden Ansprüche, **dadurch gekennzeichnet, dass** die Markersequenzen auf einem festen Träger aufgebracht werden, insbesondere einen Filter, eine Membran, ein magnetisches oder Fluorophor-markiertes Kügelchen, ein Silizium-Wafer, Glas, Metall, Kunststoff, ein Chip, ein massenspektrometrisches Target oder eine Matrix.

6. Verfahren zur Diagnose von Systemischer Lupus erythematodes, wobei
a.) mindestens eine Markersequenz einer cDNA ausgewählt aus der Gruppe SEQ 1 - 716 und / oder SEQ 1a-716a oder jeweils ein dafür kodierendes Protein oder jeweils einer Teilsequenz oder Fragment davon auf einem festen Träger aufgebracht wird und
b.) mit Körperflüssigkeit oder Gewebeauszug eines Patienten in Kontakt gebracht wird und
c.) der Nachweis einer Wechselwirkung der Körperflüssigkeit oder Gewebeauszug mit den Markersequenzen aus a.) erfolgt.

7. Verfahren zum Stratifizieren, insbesondere zur Risikostratifizierung, oder zur Therapiesteuerung eines Patienten mit Systemischer Lupus erythematodes, wobei mindestens eine Markersequenz einer cDNA ausgewählt aus der Gruppe SEQ 1 - 716 und / oder SEQ 1a-716a oder jeweils ein dafür kodierendes Protein oder jeweils einer Teilsequenz oder Fragment davon an einem zu untersuchenden Patienten bestimmt wird.

8. Verfahren nach Anspruch 7, wobei das Stratifizieren oder die Therapiesteuerung Entscheidungen zur Behandlung und Therapie des Patienten, insbesondere Hospitalisierung des Patienten, Einsatz, Wirkung und / oder Dosierung eines oder mehrerer Arzneimittel, eine therapeutische Maßnahme oder die Überwachung eines Krankheitsverlaufes sowie Therapieverlauf, Ätiologie oder Klassifizierung einer Erkrankung samt Prognose umfasst.

9. Anordnung von Markersequenzen enthaltend mindestens eine Markersequenz einer cDNA ausgewählt aus der Gruppe SEQ 1 - 716 und / oder SEQ 1a-716a oder jeweils ein dafür kodierendes Protein.

10. Anordnung nach Anspruch 9, **dadurch gekennzeichnet, dass** mindestens 2 bis 5 oder 10, vorzugsweise 30 bis 50 Markersequenzen oder 50 bis 100 oder mehr Markersequenzen enthalten sind.

11. Anordnung nach Anspruch 9, **dadurch gekennzeichnet, dass** die Markersequenzen als Clone vorliegen.

12. Assay, Proteinbiochip bestehend aus einer Anordnung nach Anspruch 9, **dadurch gekennzeichnet, dass** die Markersequenzen auf einem festen Träger aufgebracht sind.

13. Verwendung einer Anordnung nach einem der Ansprüche 9 bis 11 oder einem Assay nach Anspruch 12 zum Identifizieren und Charakterisieren einer Substanz für Systemischer Lupus erythematodes enthaltend Mittel zum Nachweis eines Bindungserfolges, **dadurch gekennzeichnet, dass** eine Anordnung oder Assay mit a.) mindestens einer zu untersuchenden Substanz in Kontakt gebracht wird und b.) ein Bindungserfolg nachgewiesen wird.

14. Verwendung einer Anordnung nach einem der Ansprüche 9 bis 11 oder einem Assay nach Anspruch 12 zum Screenen von Wirkstoffen für Systemischer Lupus erythematodes.

15. Diagnostika zur Diagnose von Systemischer Lupus erythematodes, jeweils ausgewählt aus der Gruppe SEQ 1 - 716 und / oder SEQ 1a-716a oder jeweils ein dafür kodierendes Protein oder jeweils einer Teilsequenz oder Fragment davon.

16. Target zur Behandlung und Therapie von Systemischer Lupus erythematodes, jeweils ausgewählt aus der Gruppe SEQ 1 - 716 und / oder SEQ 1a-716a oder jeweils ein dafür kodierendes Protein oder jeweils einer Teilsequenz oder Fragment davon.

17. Verwendung einer Markersequenz einer cDNA ausgewählt aus der Gruppe SEQ 1 - 716 und / oder SEQ 1a-716a oder jeweils ein dafür kodierendes Protein oder jeweils einer Teilsequenz oder Fragment davon als Affinitätsmaterial zur Durchführung einer Apherese oder Blutwäsche für Patienten mit Systemischer Lupus erythematodes.
